# EUROPEAN PATENT APPLICATION

(11) **EP 4 389 176 A1**
(43) Date of publication of application: **26.06.2024**
(21) Application number: 23218815.1
(22) Date of filing: 20.12.2023
(51) Int. Cl.: A61M 5/315

(54) **LOW INSERTION FORCE SYRINGE STOPPERS, ASSEMBLIES, AND ASSOCIATED METHODS**

(30) Priority: 20.12.2022 US 202263434008 P
(71) Applicant: W. L. Gore & Associates, Inc., Newark, DE 19711 (US)
(72) Inventor: BASHAM, Robert C., Newark, DE 19711 (US); LAROSE, Erik M., Newark, DE 19711 (US); RUSCH, Greg, Newark, DE 19711 (US)
(74) Representative: HGF

(57) **Abstract**

Plunger rod head and syringe stopper pocket designs that facilitate reduced axial insertion forces while coupling plunger rods to stoppers (e.g., for use in pre-filled syringe assemblies). Desirable axial insertion forces may be lower than the associated break loose forces between the stoppers and their associated syringe barrels, with designs still ensuring the rod head and syringe stopper are coupled and require a substantial, non-zero separation force to de-couple the components.

## Description

### BACKGROUND

Syringes typically include a barrel, a stopper positioned within the barrel, and a plunger rod to displace the stopper. The stopper is typically air and liquid impermeable and forms and air and liquid tight seal with the barrel while also possessing low-friction slidability. Low-friction slidability is important for facilitating the discharging of the liquid inside the syringe.

Assembly of a syringe typically includes coupling the plunger rod to the stopper. In a pre-filled syringe assembly method, the barrel may be filled with a therapeutic substance prior to inserting the stopper into the barrel. Once the stopper has been inserted into the barrel, the plunger rod is coupled to the stopper, either by application of an axial force to press the plunger rod head into the stopper, or via application of torque, or rotational force to screw the plunger rod head into the stopper. Examples of prior art assembly designs for such threaded, or screw type assembly include U.S. Patent 10,369,292 to W. L. Gore & Associates, Inc.

It has been observed by some that the process of creating a connection between a plunger rod and a stopper during assembly, or coupling the plunger rod and the stopper, using a screwing or a push-fit action, can distort or translate the stopper compromising the efficacy of the seal integrity or sterility of the barrel and/or therapeutic substance and/or increasing pressure in the barrel causing fluid leakage from the outlet end of the syringe. Some prior art designs purport to overcome such concerns by assembling a pre-filled syringe without coupling the plunger rod to the stopper such that the plunger rod does not need to be manipulated or forced to couple with the stopper. The concepts disclosed in U.S. Patent 11,185,635 to Novartis AG use such a non-coupled approach in an effort to address the foregoing concerns. However, following initial assembly (e.g., by dropping the plunger rod into the barrel against the stopper) the lack of coupling between the stopper and the plunger rod means that the rod is free to translate away from the stopper an unacceptable amount (e.g., completely out of the barrel) if an additional backstop is not added to the barrel.

### SUMMARY

Various embodiments relate to plunger rod head and syringe stopper pocket designs that facilitate reduced axial insertion forces while coupling plunger rods to stoppers in pre-filled syringe designs, as well as associated methods and systems. Various embodiments address axial insertion forces that are lower than break loose forces between stoppers and associated syringe barrels, while still ensuring the rod head and syringe stopper are coupled and require a substantial, non-zero separation force to de-couple the components.

According to one example ("Example 1"), a stopper for a syringe includes a body having a leading end, a trailing end, and an outer surface extending between the leading end and the trailing end, the outer surface operable to sealingly and slidably engage a barrel of a syringe; wherein the body of the stopper has an inner surface and a pocket having a first end and a second end, the pocket being defined by the inner surface and the body further having an opening into the second end of the pocket that is formed in the trailing end of the body; wherein the pocket includes a capture portion having a first diameter, a relief portion having a second diameter, and a coupling portion between the capture portion and the relief portion that has a third diameter that is less than the first and second diameters; and wherein the inner surface of the body includes one or more coupling projections corresponding to the coupling portion of the pocket.

According to another example ("Example 2"), further to Example 1, the one or more coupling projections includes a circumferential rib.

According to another example ("Example 3"), further to Example 2, the circumferential rib extends continuously about a circumference of the pocket.

According to another example ("Example 4"), further to any of Examples 1 to 3, the one or more coupling projections includes a longitudinal rib.

According to another example ("Example 5"), further to any of Examples 1 to 4, the one or more coupling projections has a leading edge and a trailing edge, and further wherein at least the leading edge is at least one of angled and rounded.

According to another example ("Example 6"), further to any of Examples 1 to 5, the first diameter approximately the same as the second diameter.

According to another example ("Example 7"), further to any of Examples 1 to 6, the third diameter is at least 10% smaller than the first diameter and/or the second diameter.

According to another example ("Example 8"), further to any of Examples 1 to 7, the first diameter differs from the third diameter by approximately 0.3 mm.

According to another example ("Example 9"), further to any of Examples 1 to 8, the second diameter differs from the third diameter by approximately 0.3 mm.

According to another example ("Example 10") a syringe includes a barrel having an outer surface, an inner surface, and a receiving chamber, the inner surface bounding the receiving chamber; a stopper positioned in the receiving chamber such that the stopper is slidably and sealingly engaged with the inner surface of the barrel, the stopper having a pocket including a capture portion having a first diameter, a relief portion having a second diameter, and a coupling portion between the capture portion and the relief portion that has a third diameter that is less than the first and second diameters, the stopper including one or more coupling projections corresponding to the coupling portion of the pocket; and a plunger rod having a head portion, a rear portion, and a rod portion extending between the head portion and the rear portion, the head portion having a tapered crown and defining retaining feature engaged with the coupling portion of the pocket to couple the plunger rod to the stopper with the head portion received in the capture portion of the stopper.

According to another example ("Example 11 "), further to Example 10, the tapered crown has a smooth surface for slidably engaging with the one or more coupling projections of the coupling portion during insertion of the plunger rod head portion into the pocket of the stopper.

According to another example ("Example 12"), further to Examples 10 or 11, the stopper and the barrel define a break loose force, and the stopper and the plunger rod are configured such that the plunger rod head portion is insertable axially into the stopper with the stopper received in the barrel at an insertion force that is less than the break loose force.

According to another example ("Example 13"), further to Example 12, the break loose force is from 2N to 20N.

According to another example ("Example 14"), further to any of Examples 10 to 13, the stopper and the plunger rod require application of a separation force in a longitudinal direction to decouple the plunger rod from the stopper.

According to another example ("Example 15"), further to Example 14 when including the features of Examples 12 or 13, the separation force is greater than the break loose force.

According to another example ("Example 16") A method of coupling a plunger rod to a stopper that has been positioned in a barrel of a syringe, the method comprising axially inserting a head portion of the plunger rod into a pocket of the stopper with an insertion force that is less than a break loose force defined between the stopper and the barrel of the syringe, wherein upon axially inserting the head portion of the plunger rod into a capture portion of the stopper the plunger rod is coupled to the stopper.

According to another example ("Example 17"), further to Example 16, inserting the head portion into the pocket includes sliding a tapered crown of the head portion of the plunger rod past one or more coupling projections corresponding to the coupling portion of the pocket in order to couple the plunger rod to the stopper.

According to another example ("Example 18"), further to Example 17, the one or more coupling projections include one or more longitudinally extending ribs and/or one or more circumferentially extending ribs.

According to another example ("Example 19"), further to any of Examples 16 to 18, each of the one or more coupling projections has a leading edge and a trailing edge, and further wherein at least the leading edge is at least one of angled and rounded, and further wherein inserting the head portion into the pocket includes sliding the head portion longitudinally past the leading edge of the one or more coupling projections.

According to another example ("Example 20"), further to Example 19, the break loose force is from 2N to 20N.

According to another example ("Example 21"), further to any of Examples 16 to 20, the stopper and the plunger rod require application of a separation force in a longitudinal direction to decouple the plunger rod from the stopper.

According to another example ("Example 22"), further to Examples 21, the separation force is greater than the break loose force.

According to another example ("Example 23"), a stopper for a syringe includes a body having a leading end, a trailing end, and an outer surface extending between the leading end and the trailing end, the outer surface operable to sealingly and slidably engage a barrel of a syringe; wherein the body of the stopper has an inner surface and a pocket having a first end and a second end, the pocket being defined by the inner surface and the body further having an opening into the second end of the pocket that is formed in the trailing end of the body; wherein the pocket includes a capture portion having a first diameter, a relief portion having a second diameter, and a coupling portion between the capture portion and the relief portion that has a third diameter that is less than the first and second diameters; and wherein the inner surface of the body includes one or more coupling recesses corresponding to the coupling portion of the pocket.

According to another example ("Example 24"), further to Example 23, the one or more coupling recesses includes a circumferential recess.

According to another example ("Example 25"), further to Example 24, the circumferential recess extends continuously about a circumference of the pocket.

According to another example ("Example 26"), further to any of Examples 23 to 25, the one or more coupling recesses includes a longitudinal recess.

According to another example ("Example 27"), further to any of Examples 23 to 26, the one or more coupling recesses has a leading edge and a trailing edge, and at least the leading edge is at least one of angled and rounded.

According to another example ("Example 28"), further to any of Examples 23 to 27, the first diameter approximately the same as the second diameter.

According to another example ("Example 29"), further to any of Examples 23 to 28, the third diameter is at least 10% greater than the first diameter and/or the second diameter.

According to another example ("Example 30"), further to any of Examples 23 to 29, wherein the first diameter differs from the third diameter by approximately 0.3 mm.

According to another example ("Example 31"), further to any of Examples 23 to 30, the second diameter differs from the third diameter by approximately 0.3 mm.

According to another example ("Example 32"), a method of coupling a plunger rod to a stopper that has been positioned in a barrel of a syringe, the method comprising axially inserting a head portion of the plunger rod into a pocket of the stopper with an insertion force that is less than a break loose force defined between the stopper and the barrel of the syringe, wherein upon axially inserting the head portion of the plunger rod into a capture portion of the stopper the plunger rod is coupled to the stopper.

According to another example ("Example 33"), further to Example 32, inserting the head portion into the pocket also includes sliding an enlarged segment of the head portion of the plunger rod into one or more coupling recesses corresponding to a coupling portion of the pocket in order to couple the plunger rod to the stopper.

According to another example ("Example 34"), further to Example 33, the one or more coupling recesses includes one or more longitudinally-extending recesses and/or one or more circumferentially-extending recesses.

According to another example ("Example 35"), further to Example 34, each of the one or more coupling recesses has a leading edge and a trailing edge, where at least the leading edge is at least one of angled and rounded, and, inserting the head portion into the pocket includes sliding the head portion longitudinally past the leading edge of the one or more coupling recesses to seat an enlarged segment of the head portion into the one or more coupling recesses.

According to another example ("Example 36"), further to any of Examples 32 to 35, the break loose force is from 2N to 20N and the stopper and the plunger rod require application of a separation force in a longitudinal direction to decouple the plunger rod from the stopper once the plunger rod is inserted into the stopper.

According to another example ("Example 37"), further to Example 36, the separation force is greater than the break loose force.

According to another example ("Example 38"), a syringe includes a barrel having an outer surface, an inner surface, and a receiving chamber, the inner surface bounding the receiving chamber; a stopper positioned in the receiving chamber such that the stopper is slidably and sealingly engaged with the inner surface of the barrel, the stopper having a pocket including a capture portion having a first diameter, a relief portion having a second diameter, and a coupling portion between the capture portion and the relief portion that has a third diameter that is greater than the first and second diameters, the stopper including one or more coupling recesses corresponding to the coupling portion of the pocket; and a plunger rod having a head portion, a rear portion, and a rod portion extending between the head portion and the rear portion, the head portion defining retaining feature engaged with the coupling recess of the pocket to couple the plunger rod to the stopper with the retaining feature received in the one or more coupling recesses of the stopper.

According to another example ("Example 39"), further to Example 38, the stopper and the barrel define a break loose force, and the stopper and the plunger rod are configured such that the plunger rod head portion is insertable axially into the stopper with the stopper received in the barrel at an insertion force that is less than the break loose force.

According to another example ("Example 40"), further to Example 39, the break loose force is from 2N to 20N.

According to another example ("Example 41"), further to any of Examples 38 to 40, the stopper and the plunger rod require application of a separation force in a longitudinal direction to decouple the plunger rod from the stopper.

According to another example ("Example 42"), further to Example 41 when including the features of Examples claims 39 or 40, the separation force is greater than the break loose force.

The foregoing Examples are just that, and should not be read to limit or otherwise narrow the scope of any of the inventive concepts otherwise provided by the instant disclosure. While multiple examples are disclosed, still other embodiments will become apparent to those skilled in the art from the following detailed description, which shows and describes illustrative examples. Accordingly, the drawings and detailed description are to be regarded as illustrative in nature rather than restrictive in nature.

### BRIEF DESCRIPTION OF THE DRAWINGS

The accompanying drawings are included to provide a further understanding of the disclosure and are incorporated in and constitute a part of this specification, illustrate embodiments, and together with the description serve to explain the principles of the disclosure.
FIG. 1 is a side view of a syringe, according to some embodiments.
FIG. 2 is a side view of a syringe, with a plunger rod and a stopper in a disassembled, or uncoupled state, according to some embodiments.
FIGS. 3A and 3B are longitudinal sections showing prior art stopper and plunger rod coupling features.
FIGS. 4A and 4B show a stopper, a stopper assembled to a plunger rod, according to some embodiments.
FIGS. 5 to 15B show various stopper pocket designs for the syringe of FIGS. 1 and 2, according to some embodiments.
FIGS. 16 to 18B show various plunger rod designs for the syringe of FIGS. 1 and 2, according to some embodiments.

### DETAILED DESCRIPTION

### Definitions and Terminology

This disclosure is not meant to be read in a restrictive manner. For example, the terminology used in the application should be read broadly in the context of the meaning those in the field would attribute such terminology.

The use of headings is provided for ease of review of the description only, and are not meant to segregate or otherwise designate that concepts under one heading are inapplicable or otherwise unrelated to concepts under another heading. In fact, the opposite is intended and the description is meant to be read and interpreted as a whole, with various features and aspects of certain embodiments being applicable across and applicable to the various other embodiments described herein.

With respect to terminology of inexactitude, the terms "about" and "approximately" may be used, interchangeably, to refer to a measurement that includes the stated measurement and that also includes any measurements that are reasonably close to the stated measurement. Measurements that are reasonably close to the stated measurement deviate from the stated measurement by a reasonably small amount as understood and readily ascertained by individuals having ordinary skill in the relevant arts. Such deviations may be attributable to measurement error, differences in measurement and/or manufacturing equipment calibration, human error in reading and/or setting measurements, minor adjustments made to optimize performance and/or structural parameters in view of differences in measurements associated with other components, particular implementation scenarios, imprecise adjustment and/or manipulation of objects by a person or machine, and/or the like, for example. In the event it is determined that individuals having ordinary skill in the relevant arts would not readily ascertain values for such reasonably small differences, the terms "about" and "approximately" can be understood to mean plus or minus 10% of the stated value.

As used herein, the terms "elastic" and "elastomeric" refer to a material property understood with reference to stoppers employed in injector devices (e.g., in FDA-approved applications) and relates to the tendency of a material to spontaneously revert back, or recover, toward its pre-deformation shape after being dimensionally deformed (e.g., contracted, dilated, distorted, or the like).

As used herein, the term "syringe" is meant to be inclusive of any of a variety devices that include a stopper received in a barrel and an actuation mechanism configured to displace the stopper within the barrel to eject, or deliver contents held in the barrel from within the barrel. The concepts disclosed herein may be utilized in conjunction with a 0.5 mL to a 20 mL syringe, for example, and may also be appropriately scaled to smaller or larger syringes.

As used herein, the term "proximal" means closer to the operator end of a device (e.g., plunger rod end) while the term distal means further away from the operator than proximal (e.g., piercing element end).

As used herein, the term "rotate" and the like (e.g., "rotation") is meant to denote circumferentially-oriented motion.

As used herein, the term "axial" insertion, translation, movement, and the like is meant to denote longitudinally-oriented motion.

As used herein, the terms "silicone" and "silicone oil" may be used interchangeably herein.

As used herein, the term "substantially free" is meant to denote an unquantifiable or trace amount of the identified substance (e.g., silicone, silicone oil, or other lubricant), or that there is not any amount intentionally added to the system (e.g., no silicone oil intentionally added to an injector device, such as the barrel or stopper).

As used herein, the term "sealing rib" means a rib of a stopper that is in contact with an inner surface a barrel to prevent the passage of air or other contaminants into the barrel or the barrel contents from exiting the barrel.

As used herein, the term "non-sealing rib" is meant to denote a rib that either does not contact an inner surface of a syringe barrel or contacts the inner surface of the barrel such that air (or other contaminants) and/or barrel contents may pass therethrough.

As used herein, the term "longitudinally extending" is meant to connote a feature having a greater dimension in the axial, or longitudinal direction, than the transverse (e.g., circumferential) direction.

As used herein, the term "circumferentially extending" is meant to connote a feature having a greater dimension in the transverse (e.g., circumferential) direction than the axial, or longitudinal direction.

As used herein, the term "break loose force" is meant to denote the force required to initiate relative movement between a stopper and a syringe barrel after the stopper is disposed in the syringe barrel.

### Description of Various Embodiments

Persons skilled in the art will readily appreciate that various aspects of the present disclosure can be realized by any number of methods and apparatuses configured to perform the intended functions. It should also be noted that the accompanying drawing figures referred to herein are not necessarily drawn to scale, and may be exaggerated to illustrate various aspects of the present disclosure, and in at least that regard, the drawing figures should not be construed as limiting.

Various concepts in this description relate to plunger rod head and syringe stopper pocket designs that facilitate reduced axial insertion forces while coupling plunger rods to stoppers (e.g., for use in pre-filled syringe assemblies). Desirable axial insertion forces may be lower than the associated break loose forces between the stoppers and their associated syringe barrels, with designs still ensuring the plunger rod head portion and syringe stopper are coupled and require a substantial, non-zero separation force to de-couple the components. Generally, the coupling, or engagement between the stopper and the plunger rods head portions are non-threaded engagements. That is, the components are configured to be assembled by axial insertion methods, rather than threading the components together. In different terms at least one of the stopper pocket and the plunger rod head portion is non-threaded, and preferably both of the stopper pocket and the plunger rod head portion are non-threaded, or do not include complementary thread features for threaded engagement between the components.

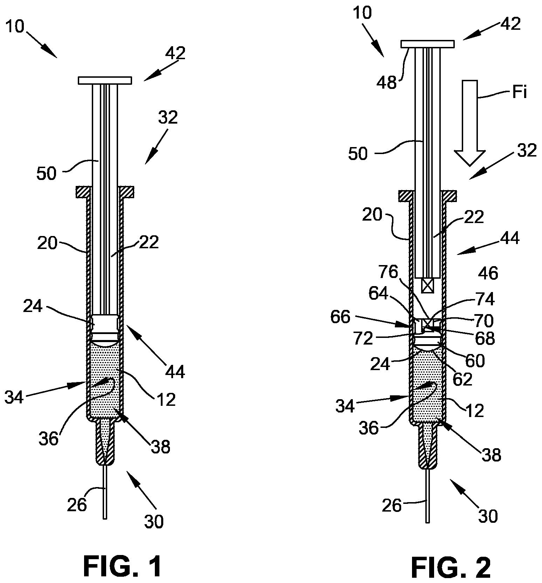

FIG. 1 shows a syringe 10, according to some examples. The syringe 10 is generally operable to deliver contents 12 of the syringe 10 by pressurizing the contents 12 such that they are expelled from the syringe 10. The syringe contents 12 may include a therapeutic agent (e.g., pharmaceutical agent) delivered to a patient in association with a medical treatment. The syringe 10 can be referenced more generally as an injector, or injection device and, although the syringe 10 may be configured as shown, the principles described herein may be applicable to other configurations for the syringe 10, including auto-injector configurations.

As shown, the syringe 10 includes a barrel 20, also described as a cartridge tube, a plunger rod 22, and a stopper 24. The syringe 10 may also include a piercing element 26, though it is also within the scope of the present disclosure for the syringe 10 to be a "needleless" device, such as those terminated with a Luer-Lok^{™} system (not shown).

In some embodiments, the barrel 20 includes a distal end 30, a proximal end 32, an outer surface 34, and an inner surface 36 that bounds, or otherwise defines a receiving chamber 38 for receiving the contents 12. The barrel 20 may be formed of a variety of materials, including relatively hard materials. Some examples of suitable materials include glass material (e.g., borosilicate glass), ceramic material, polymeric material (e.g., polypropylene, polyethylene, and copolymers thereof, cyclic olefin polymers, and cyclic olefin copolymers), metallic material, and combinations thereof. In some embodiments, the barrel 20 includes some amount of lubricant (not shown) present on the inner surface 36 of barrel 20. In other embodiments, the barrel 20 is lubricant free, or substantially free of lubricants. As shown, the contents 12 may be pre-filled in the barrel 20 as part of a syringe assembly process (i.e., as a pre-filled syringe), however it is also contemplated that in other applications the contents 12 may be drawn into the barrel 20 following assembly of the syringe 10. The inner diameter of the syringe barrel 20 can be any of a variety of values, such as from about 5mm to about 20mm, for example. In one example, the barrel 20 corresponds to a 0.5ml syringe, and has a diameter of 6.85mm ± 0.1mm. In another example, the barrel 20 corresponds to a 20ml syringe and has a diameter of 19.05mm ± 0.2mm.

The plunger rod 22 of the syringe 10 is movable within the barrel 20 to discharge the contents 12 by moving the stopper 24 towards the distal end 30 of the barrel 20. Though shown pre-filled in FIG. 1, it is contemplated that in other applications the barrel 20 may be charged, or filled, by drawing the stopper 24 in the proximal direction. FIG. 2 shows the syringe 10 in a partially assembled state and FIG. 1 shows the syringe 10 in a fully assembled state. Some assembly methods include disposing the stopper 24 in the barrel 20 with the contents 12 received in the barrel 20 as shown in FIG. 2. The plunger rod 22 can then be assembled to the stopper by employing an axial insertion force Fi on the plunger rod 22. As shown in FIG. 2, the plunger rod 22 includes a proximal end 42, a distal end 44, a head portion 46 at the distal end 44, a rear portion 48 at the proximal end 42, and a rod portion 50 extending between the head portion 46 and the rear portion 48. For reference, the head portion 46 is shown generically with a box and an "X" as several different configurations for the head portion 46 are contemplated and are subsequently described in further detail. In general terms, the head portion 46 is configured to couple the plunger rod 22 to the stopper 24.

As shown in FIG. 2, the stopper 24 is positioned in the receiving chamber 38 such that the stopper 24 is slidably and sealingly engaged with the inner surface 36 of the barrel 20. The stopper 24 has a body 60 having a leading end 62, a trailing end 64, and an outer surface 66 extending between the leading end 62 and the trailing end 64, the outer surface 66 operable to sealingly and slidably engage the barrel 20 of the syringe 10. The body 60 further has an inner surface 68 and a pocket 70 having a first end 72 and a second end 74, the pocket 70 being defined by the inner surface 68. The body 60 has an opening 76 into the second end 74 of the pocket 70 that is formed in the trailing end 64 of the body 60. Similarly to the head portion 46 of the plunger rod 22, the pocket 70 is shown in generalized form with a box and an "X" as several different configurations for the pocket 70 are contemplated and are subsequently described in greater detail. The outer surface 66 of the stopper 24 optionally defines one or more ridges, ribs, valleys, recesses, or other features for helping the stopper 24 sealingly and slidably engage with the inner surface 36 of the barrel 20.

The body 60 of the stopper 24 is optionally formed of an elastomeric material. The stopper 24 may also include a thin film cover formed of a polymeric material, such as a fluoropolymer material, including PTFE, ePTFE, and variants thereof. Examples of suitable stopper and film cover materials may be found in U.S. Patent 10,471,211 to W. L. Gore & Associates, Inc. In some examples, the thin film cover can serve as a solid lubricant. The thin film cover may be provided in addition to or as an alternative to a lubricant (e.g., silicone oil) provided in the barrel 20. The stopper 60 should generally have low air and liquid permeability to minimize liquid leakage within the barrel 20 and the introduction of air between the stopper 24 and the inner surface 68 of the barrel 20 when charging and/or discharging the contents 12 of the syringe 10. The overall diameter of the stopper 24 can be any of a variety of values, but in some examples for a 0.5mL barrel is from 5.2mm to 5.5mm OD +/-0.1 mm and for a 20mL barrel is from 19.9mm to 21mm OD +/- 0.15mm, although a variety of dimensions are contemplated.

As shown in FIG. 1, the stopper 24 contacts the inner surface 36 of the barrel 20 via one or more sealing ribs, although any number of sealing ribs and/or non-sealing ribs may be present on the stopper 24. Generally, a break loose force is required to be applied in order to initiate movement of the stopper 24 within the barrel 20. Break loose forces are often measured as "wet" or "dry" values (i.e., filled with liquid contents, such as saline or water vs. empty). Generally, such measurements are taken for a pre-filled syringe ("wet") rather than an empty syringe ("dry"). Break loose forces for pre-filled syringes are typically 20N or less. In various examples, the break loose force is less than 15N. In some pre-filled syringes, the break loose force is less than about 11N or less than 9N, less than 7N, less than 5N or between about 2N to 5N. In some examples, for a 0.5mL syringe, the targeted break loose force (pre-filled, or "wet") by design is approximately 4N, with an average range of between 2N and 8N. Break force, or break loose force, may be assessed using methods similar to those described in ISO 11040-8, Section 6. Reference may also be had to ISO 11040-4, Appendix E for glide force measurement techniques, which may be used to assess break loose force. The break loose force may be measured at a stopper travelling speed of 250 mm/min within the barrel 20. Patent U.S. Patent 10,369,292 to W. L. Gore & Associates, Inc. describes various expected break loose forces encountered in pre-filled syringes. U.S. Patent 9,220,631 to Juergen et al. also describes various expected break loose forces encountered in pre-filled syringes.

As shown, the piercing element 26 of the syringe 10 is coupled to the distal end 30 of the barrel 20. The piercing element 26 is optional, as the syringe 10 may be "needleless" and/or not coupled to the barrel 20 as desired (e.g., as may be the case in autoinjector devices). When present, the piercing element 26 may be configured to pierce a patient's skin to inject the contents 12 into the patient.

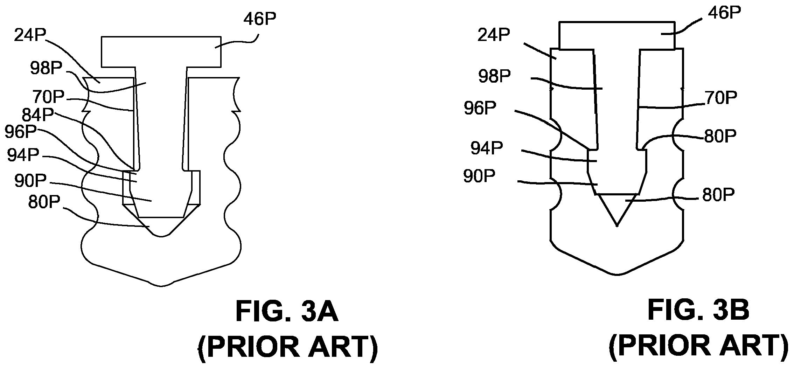

FIGS. 3A and 3B show a prior art head portion design 46P for the head portion 46 of the plunger rod 22 (FIG. 1) and a prior art stopper design 24P for the stopper 24. The views of FIGS. 3A and 3B are longitudinal sections of the stopper 24P. The head portion 46P is configured to be axially inserted into a pocket 70P of the stopper 24P to couple the head portion 46P to the stopper 24P. FIG. 3A shows the head portion 46P in the pocket 70P without the stopper 24P in a compressed state as it would otherwise be when placed in a syringe barrel 20. In different terms, FIG. 3A shows the stopper 24P in a relaxed state. FIG. 3B is representative of the shape and interface between the stopper 24P and the pocket 70P when the stopper 24P is in a compressed state as received in the barrel 20 of the syringe 10. Thus, in terms of the actual interaction between the head portion 46P and the stopper 24P when the syringe 10 is assembled, FIG. 3B is more representative that FIG. 3A.

As shown, the pocket 70P includes a capture portion 80P having an enlarged diameter and a coupling portion 84P having a reduced diameter relative to the enlarged diameter of the coupling portion 84P. As shown, the capture portion 80P has a constant diameter and is cylindrical and then tapers distally, or is conical in shape. The capture portion 80P extends for a remainder of the pocket 70P, has a constant diameter, and is cylindrical in shape.

The head portion 46P, in turn, has a tapered crown 90P having a first diameter, an enlarged segment 94P defining a retaining feature 96P where the enlarged segment 94P reduces in diameter to a reduced segment 98P, the reduced segment 98P having a smaller diameter than the enlarged segment 94P. In one embodiment, the head portion 46P defines an OD diameter of approximately 2.06mm at the retaining feature 96P. As shown in FIG. 3B, the enlarged segment 94P engages with the capture portion 80P, the retaining feature 96P engages with the coupling portion 84P, and the reduced segment 98P also engages with the coupling portion 84P. These various engagements couple the stopper 24 to the head portion 46P, but require significant axial insertion forces Fi in order to insert the head portion 46 into the pocket 70P.

With regard to the interaction between the prior art pocket 70P and head portion 46P, it has been observed that during insertion, the stopper 24P may move or be displaced in the syringe 10 as the axial insertion force Fi on the plunger rod 22 that is required to insert the head portion 46P into the pocket 70P exceeds the break loose force between the stopper 24P and the barrel 20. Once the break loose force has been exceeded, the prior art stopper design 24P moves distally in the barrel 20, risking ejection of the contents 12 and/or other unwanted effects (e.g., loss of seal).

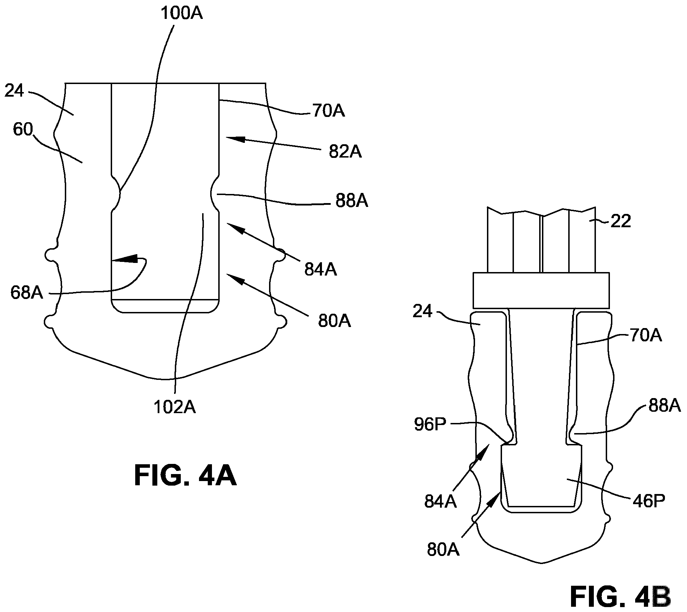

FIGS. 4A to 15 are illustrative of modified stopper designs usable with head portions similar to the head portion 46P, or alternative head portion designs that facilitate assembly without requiring high axial insertion forces Fi for rod coupling otherwise present in prior art designs. In different terms, the various stopper pocket designs provided in FIGS. 4A to 15C facilitate rod insertion to couple the stopper rod 22 to the stopper 24 without exceeding break loose forces between the stopper 24 and the barrel 20.

FIGS. 4A and 4B are longitudinal sections showing the stopper 24 of FIGS. 1 and 2 provided with a pocket 70A that promotes lower insertion forces Fi. FIG. 4A shows the stopper 24 in a relaxed, or uncompressed state. And, FIG. 4B shows the stopper 24 in the compressed state (as it would generally appear within the barrel 20) with the head portion 46P inserted into the pocket 70A. The various features of the stopper 24 previously described with reference to FIG. 1 are applicable to the design of FIGS. 4A and 4B, with the exception of the additional detail shown and described for the pocket 70A, and thus are left from further discussion. For reference, additional examples of pocket designs are provided and use similar reference numbers to indicate similar features in the description that follows and/or the drawings. For example, a pocket 70B is also described and shown, and similar features to those of the pocket 70A are indicated with the same reference number as the pocket 70A followed by a "B" either in the description and/or in the drawings to indicate the presence of similar features, having similar characteristics, to those described above.

As shown in FIG. 4A, the pocket 70A includes a capture portion 80A having a first diameter, a relief portion 82A having a second diameter, and a coupling portion 84A between the capture portion 80A and the relief portion 82A that has a third diameter that is less than the first and second diameters. An inner surface 68A of the body 60 includes one or more coupling projections 88A corresponding to the coupling portion 84A of the pocket 70A.

As shown, the first diameter is approximately the same as the second diameter. In some embodiments, the third diameter is at least 1% smaller than the first diameter and/or the second diameter. In one example, the first and second diameters are about 2.4mm ID with a 2.05mm ID for the third diameter prior to compression within the barrel 20. In some other examples, the one or more coupling projections protrude about 0.175mm (on each side) relative to the surrounding portions of the stopper 24.

As shown in FIG. 4A, the one or more coupling projections 88A includes a circumferential rib. The circumferential rib optionally extends continuously about a circumference of the pocket 70A. The one or more coupling projections 88A additionally or alternatively includes a longitudinal rib (not shown), such as one of those described herein in association with other pocket designs. As shown in FIG. 4A, the coupling projection 88A has a leading edge 100A and a trailing edge 102A. In some embodiments, at least the leading edge 100A is at least one of angled and rounded. As shown in FIG. 4A, the leading edge 100A and the trailing edge 102A are each rounded. Rounding, or angling, of the leading edge 100A can help reduce the axial insertion force Fi required to insert the plunger rod 22 in the stopper 24.

FIG. 4B shows the stopper 24 compressed, and as such shows the pocket 70A as it would generally appear when interacting with the head portion 46 of the plunger rod 22, such as that according to the design of the head portion 46P. As shown, the retaining feature 96P is engaged with the coupling portion 84A of the pocket 70A to couple the plunger rod 22 to the stopper 24 with the head portion 46P received in the capture portion 80A of the stopper 24.

During insertion into the pocket 70A, the tapered crown 90P slidably engages with the coupling projection 88A of the coupling portion 84A during insertion of the head portion 46P into the pocket 70A of the stopper 24. The stopper 24 including the pocket 70A is configured such that the head portion 46P is insertable axially into the stopper 24 with the stopper 24 received in the barrel 20 (FIG. 1) at an insertion force Fi that is less than the break loose force (e.g., where the break loose force is from 2N to 20N). Following insertion, the head portion 46P is captured in the capture portion 80A of the pocket 70A.

In order to de-couple the head portion 46P from the pocket 70A, a non-zero separation force must be applied in a longitudinal direction between the stopper 24 and the plunger rod 22 in order to separate the components by withdrawing the retaining feature 96P back over the coupling portion 84A of the pocket 70A. As shown, the smooth surface of the tapered crown facilitates insertion of the retaining feature 96P over the coupling projection 88A, lowering the requisite axial insertion force, while the relatively hard, or angled edge of the retaining feature 96P inhibits retraction back over the coupling projection 88A once the head portion 46P is seated in the capture portion 80A. In some embodiments, the separation force is greater than 2N. In some embodiments, the separation force exceeds, or is greater than, the break loose force. For reference, an axial insertion force is clearly distinguishable from a rotational, or torsional insertion force, which would be required for a threaded engagement. The embodiments described herein are provided for axial insertion assembly, rather than threaded, screw-in assembly methods. For reference, the embodiments described herein may be utilized with equipment used to attach plunger rods to stoppers that include torsional/threading movement (the barrel 20 and stopper 24 spin onto the plunger rod 22 along with axial movement). And, this torsional movement may be present even in "push-in" style plunger rods similar to those described herein. Regardless, in various examples, the plunger rod 22, and more specifically the head portion 46, is pushed axially past the one or more coupling projections of the stopper 24, as opposed to being threaded past such coupling projections.

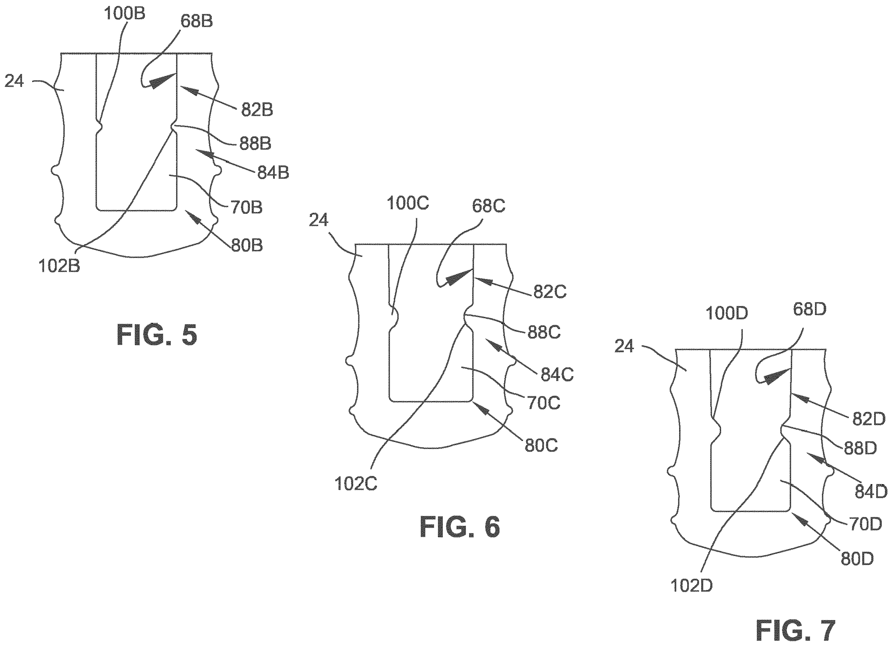

FIGS. 5 to 10A are longitudinal sections illustrating several design variations for the pocket 70 configured to achieve a similar result of a lower axial insertion force Fi than an associated break loose force of the stopper 24.

FIG. 5 is a longitudinal section showing the stopper 24 of FIGS. 1 and 2 provided with a pocket 70B that also promotes lower insertion forces Fi than the pocket 70P. FIG. 5 illustrates one or more coupling projections 88B that include a circumferential rib of smaller size (narrower) than that of the coupling projection 88A (FIG. 4A). The rib defines approximately the same inner diameter as the rib of the pocket 70A. From this (and the additional examples that follow), it should be understood that various dimensions for the coupling projections are contemplated. Similarly to the coupling projection 88A, the circumferential rib extends continuously about a circumference of the pocket 70B. Otherwise, the pocket 70B operates similarly to the pocket 70A, although it is expected on average that the axial insertion force Fi will be lower with the pocket 70B than the pocket 70A, as will be the associated separation force, due to the smaller profile for the coupling projection 88A.

FIG. 6 is a longitudinal section showing the stopper 24 of FIGS. 1 and 2 provided with a pocket 70C that promotes lower insertion forces Fi than the pocket 70P. FIG. 6 is illustrates one or more coupling projections 88C that include a circumferential rib of larger size (wider) than that of the coupling projections 88A (FIG. 4A) and 88B (FIG. 5). Similarly to the coupling projections 88A and 88B, the circumferential rib extends continuously about a circumference of the pocket 70C. Otherwise, the pocket 70C operates similarly to the pockets 70A and 70B, although the axial insertion force Fi is expected to be higher on average with the pocket 70C than the pockets 70A and 70B, as will be the associated separation forces, due to the larger (wider) profile for the coupling projection 88A.

FIG. 7 is a longitudinal section showing the stopper 24 of FIGS. 1 and 2 provided with a pocket 70D that promotes lower insertion forces Fi than the pocket 70P. FIG. 7 is meant to illustrate one or more coupling projections 88D that includes a circumferential rib of larger size (wider) than that of the coupling projections 88A (FIG. 4A), 88B (FIG. 5), and 88C (FIG. 6). Similarly to the coupling projections 88A, 88B, and 88C, the circumferential rib extends continuously about a circumference of the pocket 70D. Otherwise, the pocket 70D operates similarly to the pockets 70A, 70B, 70C. As shown, the coupling projection 88D has a relatively flat peak, or apex. By flattening the design of the coupling projection 88D. Notably, this same flattening can be applied to the narrower peak, or apex of the coupling projection 88A.

Flattening of the one or more coupling projections 88 may enhance stiffness of the coupling projection, reducing insertion force, and/or to help increase separation force. In terms of other variations, thickening and/or lengthening the coupling projections may help with manufacturing processes, strength or robustness of the coupling projection, and/or increase separation forces between the stopper 24 and the plunger rod 22. In some examples, larger radii features exhibit greater stiffness than relatively smaller radii features, which may be more prone to tearing during molding, coupling with the plunger rod 22, or decoupling from the plunger rod 22.

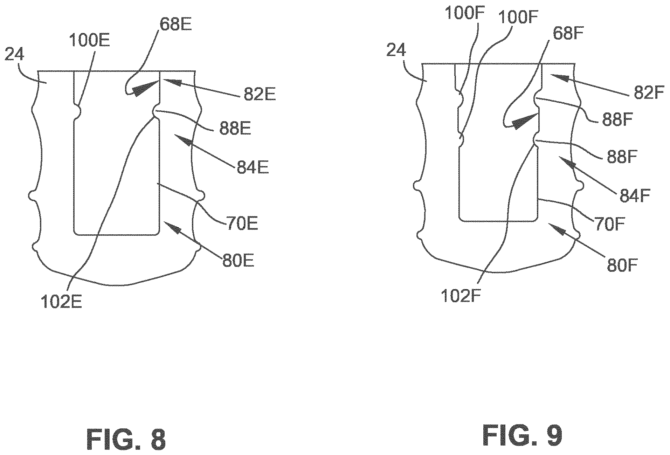

FIG. 8 is a longitudinal section showing the stopper 24 of FIGS. 1 and 2 provided with a pocket 70E that promotes lower insertion forces Fi than the pocket 70P. FIG. 7 is meant to illustrate one or more coupling projections 88E that includes a circumferential rib of any of a variety of sizes or configurations (narrower, wider, less pronounced, and more pronounced, rounded peak, flattened peak, etc.). However, as shown, the coupling project 88E is positioned more proximally than the coupling projections 88A, 88B, 88C, 88D. Similarly to the foregoing coupling projections, the circumferential rib extends continuously about a circumference of the pocket 70E. The pocket 70E operates similarly to the foregoing pockets, with the exception that the capture portion 80E is substantially longer than in other embodiments, thus giving the head portion 46P (not shown) more distal-proximal play, or potential room to displace, relative to the other embodiments which include the coupling projection directly engaging the retaining feature 96P (not shown) when the head portion 46P is fully inserted into the capture portion 80E. This feature may promote a desired amount of proximal sliding, or displacement, before the retaining feature 96P engages the coupling projection 88E to increasingly resist any further movement between the components. In this manner, the stopper 24 and the plunger rod 22 may remain coupled, and only separable upon application of a separation force similar to those previously described, while also permitting some relative axial displacement between the two components.

FIG. 9 is a longitudinal section showing the stopper 24 of FIGS. 1 and 2 provided with a pocket 70F that promotes lower insertion forces Fi than the pocket 70P during coupling of the stopper 24 and the plunger rod 22. FIG. 9 illustrates a plurality of coupling projections 88F that include a plurality of circumferential ribs of any of the foregoing sizes or configurations (narrower, wider, less or more pronounced, rounded peak, flattened peak, or other modifications). As shown, the plurality of coupling projections 88E include one rib positioned more proximally and a second rib positioned more distally (e.g., in a similar position as the coupling projections 88A, 88B, 88C, 88D). In at least this manner, at least one of the coupling projections 88F directly engages the retaining feature 96P (not shown) when the head portion 46P (not shown) is fully inserted into the capture portion 80F. This feature may promote a higher degree of engagement (e.g., a higher separation force) without substantially increasing the concomitant axial insertion force Fi required to insert the head portion 46P to couple the stopper 24 and the plunger rod 22.

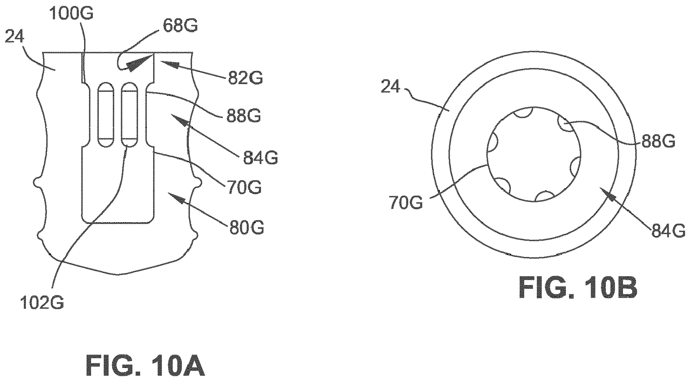

FIG. 10A is a longitudinal section showing the stopper 24 of FIGS. 1 and 2 provided with a pocket 70G that promotes lower insertion forces Fi than the pocket 70P during coupling of the stopper 24 and the plunger rod 22. FIG. 10B is an end view into the pocket 70G illustrating the features thereof from a perspective different from that of FIG. 10A. As shown, the pocket 70G includes one or more coupling projections 88G that include a plurality of longitudinal ribs having any of the foregoing sizes or configurations (narrower, wider, more or less pronounced, rounded peak, flattened peak, or other modifications). As shown, the plurality of coupling projections 88G include a plurality of longitudinal ribs circumferentially-spaced from one another. As shown, the ribs are equidistant from one another and are each similarly configured (sized and shaped), it should be understood that those configuration variables may be modified as desired, including the number of ribs (e.g., six are shown).

Each of the ribs has a leading edge 100G and a trailing edge 102G. As previously referenced, the leading edge 100G can be at least one of angled and rounded. As shown, the leading edge 100G and the trailing edge 102G of each rib are each rounded. Rounding, or angling, of the leading edge 100G can help reduce the axial insertion force Fi required to insert the plunger rod 22 in the stopper 24. In various examples, the trailing edge 102G of each coupling projection 88G is positioned to directly engage the retaining feature 96P (not shown) when the head portion 46P (not shown) is fully inserted into the capture portion 80G to couple the stopper 24 and the plunger rod 22.

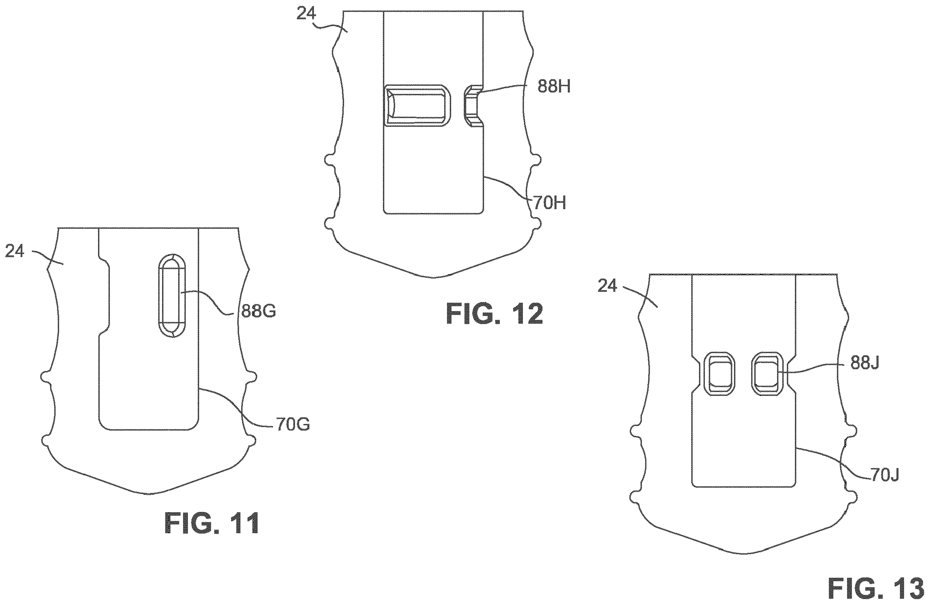

FIGS. 11 to 13 provide additional examples of coupling projection designs, or configurations, according to some embodiments.

For example, FIG. 11 is a longitudinal section showing the stopper 24 of FIGS. 1 and 2 provided with a modification on the number of coupling projections 70G relative to FIGS. 10A and 10B, where pocket 70G of FIG. 12 also promotes lower insertion forces Fi than that of FIGS. 10A and 10B as well as the pocket 70P during coupling of the stopper 24 and the head portion 70P. As shown in FIG. 11, the pocket 70G includes one or more coupling projections 88G that include a plurality of longitudinal ribs having any of the foregoing sizes or configurations (narrower, wider, more or less pronounced, rounded peak, flattened peak, etc.). In the example of FIG. 11, the one or more coupling projections 88G include longitudinal ribs configured similarly to those described in association with FIGS. 10A and 10B, although fewer ribs (three, as shown) are included. Generally, the number and configuration of the longitudinal ribs, or flutes, can be modified to help reduce the axial force of insertion while providing the desired separation force performance. Such longitudinal ribs, or flutes, can also assist with combined torsional and axial installation (though such installation is to still be contrasted with threaded installation). Otherwise, the pocket 70G of FIG. 11 optionally operates substantially similarly to pocket 70G of FIG. 10A, having similar features with similar functions.

Due to the incorporation of fewer of the coupling projections 70G (e.g., three of equidistant spacing) than that shown in FIGS. 10A and 10B, the pocket 70G as modified according to FIG. 11 may have a lower axial insertion force Fi as well as a lower separation force than the configuration of FIGS. 10A and 10B. In the example of FIG. 11, each of the coupling projections is spaced apart (e.g., by an equidistant spacing) by a greater distance than shown in FIG. 10A. For example, in FIG. 10A each coupling projection 88G may be separated by a width that is between 0.5x and 1.5x a width of the coupling projections 88G. As shown in FIG. 11, each coupling projection 88G may be separated circumferentially by a width that is greater than 2x a width of the coupling projections 88G.

FIG. 12 is a longitudinal section showing the stopper 24 of FIGS. 1 and 2 provided with pocket 70H that promotes lower insertion forces Fi than the pocket 70P during coupling of the stopper 24 and the head portion 46P. As shown, the pocket 70H includes one or more coupling projections 88H (e.g., three of equidistant spacing) that include a plurality of circumferentially-extending ribs having any of the foregoing sizes or configurations (narrower, wider, more or less pronounced, rounded peak, flattened peak, etc.), that are circumferentially aligned and spaced from one another. Other than the spacing between the ribs, the circumferential ribs operate substantially similarly to pocket 70D (FIG. 7) (which also includes a wider, flatter profile for the circumferential ribs), having similar features with similar functions.

FIG. 13 is another example of a longitudinal section showing the stopper 24 of FIGS. 1 and 2 provided with pocket 70J that promotes lower insertion forces Fi than the pocket 70P during coupling of the stopper 24 and the head portion 70P. As shown, the pocket 70J includes one or more coupling projections 88J similar to the coupling projections 70J of FIG. 12 but with more divisions (and thus a greater number of coupling projections 70J, such as six total) than FIG. 12. The coupling projections 70J are circumferentially aligned and spaced from one another a desired distance. Other than the relative shorter longitudinal lengths, and wider, more flat profiles than the fluted, or longitudinally-extending rib designs, the ribs shown in FIG. 13 operate substantially similarly to prior examples, having similar features with similar functions.

In terms of design, the examples of FIGS. 10A to 13 each include circumferentially discontinuous and/or circumferentially spaced, longitudinally extending coupling projections. By spacing the coupling projections about the circumference, some radial expansion or deformation of the coupling projections (or closing of the gaps between coupling projections) during insertion of the plunger rod 22 which may assist with lowering required axial insertion forces. In different terms, these fluted designs (spaced, longitudinally oriented coupling projections) or discontinuous circumferential designs (spaced, circumferentially oriented coupling projections) facilitate greater deformation of the stopper 24 at the coupling projections to reduce expected insertion forces.

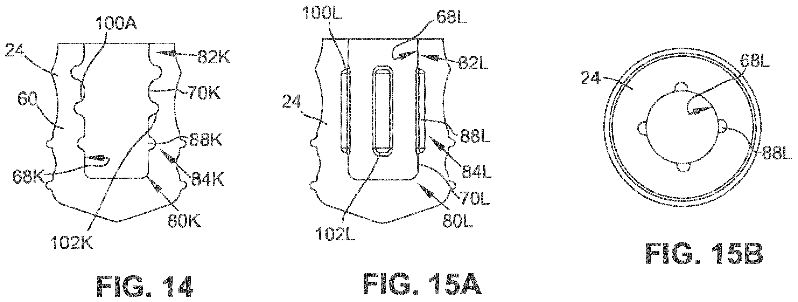

FIGS. 14 to 15B illustrate alternative designs including coupling recesses, as opposed to coupling projections, for engaging with head portion 46P, or other head portions as desired.

FIG. 14 is a longitudinal section showing the stopper 24 of FIGS. 1 and 2 provided with pocket 70K that promotes lower insertion forces Fi than the pocket 70P during coupling of the stopper 24 and the head portion 46P.

As shown in FIG. 14, the pocket 70K includes a capture portion 80K having a first diameter, a relief portion 82K having a second diameter, and a coupling portion 84K between the capture portion 80K and the relief portion 82K that has a third diameter that is greater than the first and second diameters at each of the afore-mentioned one or more recesses associated with the coupling portion 84K. In particular, an inner surface 68K of the body 60 includes one or more coupling recesses 88K corresponding to the coupling portion 84A of the pocket 70A.

As shown, the first diameter is approximately the same as the second diameter. In some embodiments, the third diameter is at least 10% greater than the first diameter and/or the second diameter. In one example, when the stopper 24 is in a compressed state in the barrel 20, the third diameter is about 2.8mm. In such an example, the stopper 24 can be paired with a plunger rod head portion having a retaining feature that defines an OD diameter of about 2.4mm, 2.6mm, or 2.8mm, for example, depending on a desired engagement between the stopper 24 and the plunger rod head portion 46. In another example, the first and second diameters are about 2.05mm OD with a 2.4mm OD for the third diameter prior to compression within the barrel 20. In some other examples, the one or more coupling projections are recessed by about 0.175mm (on each side) relative to the surrounding portions of the stopper 24.

As shown in FIG. 14, the one or more coupling recesses 88K includes one or more circumferential recesses (e.g., a generally rib-shaped recess as shown). The circumferential recess optionally extends continuously about a circumference of the pocket 70K. The one or more coupling recesses 88K additionally or alternatively includes a longitudinal recess (not shown), such as one of those described herein in association with FIGS. 15A and 15B. As shown in FIG. 14, the coupling recess 88K has a leading edge 100K and a trailing edge 102K. In some embodiments, at least the leading edge 100K is at least one of angled and rounded. As shown in FIG. 14, the leading edge 100K and the trailing edge 102K are each rounded.

FIG. 14 shows the stopper 24 uncompressed, and as such shows the pocket 70A as it would generally appear before inserted into the barrel 20 and actually interacting with the head portion 46 of the plunger rod 22.

During insertion into the pocket 70K, the tapered crown 90P is slidably received within the one or more coupling recesses 88K of the coupling portion 84K following insertion of the head portion 46P into the pocket 70K of the stopper 24. In particular, the retaining feature 96P of the head portion 46P may be received in one of the one or more coupling recesses 88K. The stopper 24 including the pocket 70K is configured such that the head portion 46P is insertable axially into the stopper 24 with the stopper 24 received in the barrel 20 (FIG. 1) at an insertion force Fi that is less than the break loose force (e.g., where the break loose force is from 2N to 20N). Following insertion, the head portion 46P may be captured in the capture portion 80K of the pocket 70K.

In order to de-couple the head portion 46P from the pocket 70K, a non-zero separation force must be applied in a longitudinal direction between the stopper 24 and the plunger rod 22 in order to separate the components by withdrawing the retaining feature 96P from the one or more coupling recesses 88K of the pocket 70K. As shown, the smooth surface of the tapered crown facilitates insertion of the retaining feature 96P into the coupling portion 84K and into the one or more coupling recesses 88K, lowering the requisite axial insertion force, while the relatively hard, or angled edge of the retaining feature 96P inhibits retraction back from the coupling portion 84K once the head portion 46P is seated in the capture portion 80A. As previously described, in some embodiments the separation force is greater than 2N. In some embodiments, the separation force exceeds, or is greater than, the break loose force. For reference, an axial insertion force is clearly distinguishable from a rotational, or torsional insertion force, which would be required for a threaded engagement.

As previously referenced, the embodiments described herein are provided for axial insertion assembly, rather than threaded, screw-in assembly methods. Again, the embodiments described herein may be utilized with equipment used to attach plunger rods to stoppers that include torsional/threading movement (the barrel 20 and stopper 24 spin onto the plunger rod 22 along with axial movement). And, this torsional movement may be present even in "push-in" style plunger rods similar to those described herein. Regardless, in various examples, the plunger rod 22, and more specifically the head portion 46, is pushed axially past the one or more coupling projections of the stopper 24, as opposed to being threaded past such coupling projections.

FIGS. 15A and 15B illustrate longitudinal sections of a longitudinally oriented, or a fluted recess design variation for the pocket 70 that is also configured to achieve a similar result of a lower axial insertion force Fi than an associated break loose force of the stopper 24.

FIG. 15A is a longitudinal section showing the stopper 24 of FIGS. 1 and 2 provided with a pocket 70L that also promotes lower insertion forces Fi than the pocket 70P. Other than the longitudinal orientation of the recesses, the features of the pocket 70L are substantially similar to those of the pocket 70K. FIG. 15A illustrates one or more coupling recesses 88L that include one or more longitudinally-extending coupling recesses (e.g., rib shaped recess). The plurality of longitudinal recesses having any of the foregoing sizes or configurations (narrower, wider, more or less pronounced, rounded peak, flattened peak, or other modifications). As shown, the plurality of coupling recesses 88L include a plurality of longitudinal rib-shaped depressions, or recesses circumferentially-spaced from one another. As shown, the recesses are equidistant from one another and are each similarly configured (sized and shaped), it should be understood that those configuration variables may be modified as desired, including the number of recesses (e.g., four are present in the design of FIG. 15A). FIG. 15B is a transverse cross-section through the one or more coupling recesses 88L showing how the recesses are clocked, or spaced from one another.

Each of the coupling recesses 88L has a leading edge 100L and a trailing edge 102L. As previously referenced, the leading edge 100L can be at least one of angled and rounded. As shown, the leading edge 100L and the trailing edge 102L of each recess are each rounded. In various examples, the leading edge 100L and/or the trailing edge 102L of each coupling recess 88L may be positioned to directly engage the plunger rod 22 when the plunger rod 22 is fully inserted into the capture portion 80L to couple the stopper 24 and the plunger rod 22.

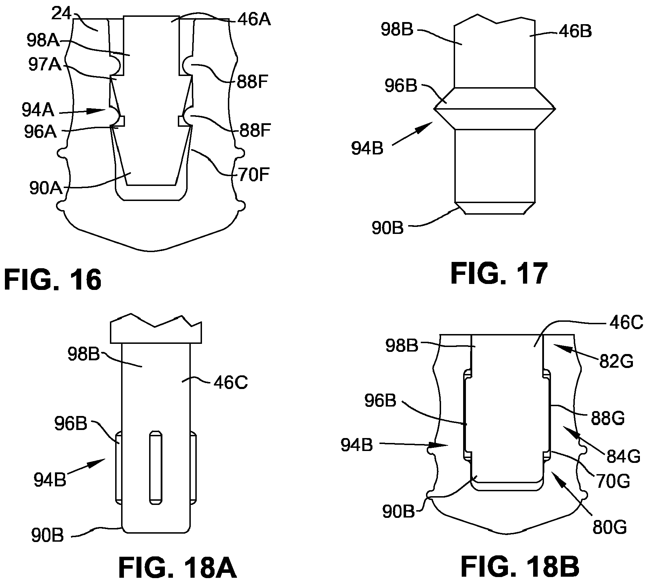

FIGS. 16 to 18B illustrate alternative head portion designs for the plunger rod 22. Any of the foregoing stopper designs may be utilized with the head portion designs shown in FIGS. 16 to 18.

FIG. 16 is a longitudinal section showing head portion 46A for the plunger rod 22 (FIG. 1), the head portion 46A promoting lower insertion forces Fi when coupling the plunger rod 22 to the stopper 24. FIG. 16 shows a small portion of the stopper 24 around the pocket 70F (see FIG. 9 and associated description) in a compressed state. In one example, the head portion 46A has a maximum outer diameter OD at the retention feature 96A of approximately 2.06mm, 2.1mm, 2,2mm, or 2,3mm for example, depending on a desired engagement with the corresponding design for the stopper 24, and specifically the requisite axial insertion force Fi and separation force.

The head portion 46A is configured to be axially inserted into a pocket (e.g., pocket 70F) of the stopper 24 to couple the head portion 46A to the stopper 24. FIG. 16 shows the head portion 46A in the pocket 70F with the stopper 24 in a compressed state as it would otherwise be when placed in a syringe barrel 20.

The head portion 46A has a tapered crown 90A having a first diameter and terminating with a flat end and extending to a first enlarged segment 94A that sharply reduces in diameter to define a first retaining feature 96A and a second tapered segment that increases in diameter proximally to and sharply reduces in diameter at a proximal engagement segment 98A to define a second retaining feature 97A. The proximal engagement segment 98A has a smaller diameter than the first enlarged segment 94A and the second enlarged segment 98A.

As indicated in FIG. 16 (with the stopper 24 shown in the compressed state), the first retaining feature 96A and the second retaining feature 97A are configured to engage with the plurality of coupling projections 88F of the pocket 70F. These various engagements couple the stopper 24 to the head portion 46A, but require lower axial insertion forces Fi in order to insert the head portion 46A into the pocket 70A (e.g., lower than a break loose force between the stopper 24 and the barrel 20 as previously described), while still requiring a substantial, non-zero separation force in order to de-couple the stopper 24 from the head portion 46A.

FIG. 17 is a side view showing a head portion 46B for the plunger rod 22 (FIG. 1), the head portion 46B configured to promote lower insertion forces Fi than the break loose force between the stopper 24 and the barrel 20 as previously described when coupling the plunger rod 22 to the stopper 24.

The head portion 46B is configured to be axially inserted into a pocket (e.g., pocket 70k or a variation thereof) of the stopper 24 to couple the head portion 46B to the stopper 24. As shown, the head portion 46B has a tapered crown 90B having a first diameter and terminating in a flat end, an enlarged segment 94B where the enlarged segment 94B increases in diameter to a peak and then decreases in diameter to a proximal engagement segment 98B, the proximal engagement segment 98B having a smaller diameter than the enlarged segment 94B. In contrast to the design of FIG. 16, which includes one or more sharp transitions to define retaining features, the enlarged segment 94B tapers, or increases proximally in width, or diameter to a peak, and then tapers, or decreases proximally in width, or diameter, to the proximal engagement segment 98B. The enlarged segment 94B is configured to engage with the coupling portion of a stopper, such as the coupling portion 84K (Fig. 14). For example, the enlarged segment 94B may define a retaining feature 96B engaging with one of the one or more coupling recesses 88K. Though shown with a relatively sharp apex defining the retaining feature 96B, a rounded or squared apex may also be implemented, for example. Moreover, a plurality of similar retaining features may be positioned along the length of the head portion 46B corresponding to the one or more coupling recesses 88K of the pocket 70K (FIG. 14) to facilitated coupling of the plunger rod 22 to the stopper 24. These various engagements couple the stopper 24 to the head portion 46B, but require lower axial insertion forces Fi than the break loose force between the barrel 20 and the stopper 24 in order to insert the head portion 46B into the pocket 70 (e.g., the pocket 70K), while still requiring a substantial, non-zero separation force in order to de-couple the stopper 24 from the head portion 46B.

FIG. 18A is a side view showing head portion 46C for the plunger rod 22, the head portion 46C promoting lower insertion forces Fi when coupling the plunger rod 22 to the stopper 24 than the break loose force exhibited between the barrel 20 and the stopper 24. As shown, the head portion 46C includes one or more (e.g., four) retaining features 96C in the form of surface projections that define longitudinally extending ribs. The one or more retaining features 96C may be configured, or otherwise sized and shaped, in a complementary fashion to a coupling portion of the stopper 24, such as the one or more coupling recesses 88G of the pocket 70G. In different terms, the one or more retaining features 96C may define a complementary fit with the one or more coupling recesses 88G.

The head portion 46C may be configured to be axially inserted into a pocket (e.g., pocket 70G, FIGS. 15A and 15B) of the stopper 24 to couple the head portion 46C to the stopper 24. For example, FIG. 18B is a longitudinal section showing the head portion 46C in the pocket 70G with the stopper 24 shown in a compressed state as it would otherwise be when placed in a syringe barrel 20.

As shown, the head portion 46C terminates in a cylindrical segment with a flat end and has an enlarged segment 94C where the enlarged segment 94C is increased in diameter via the longitudinal surface projections defining the one or more retaining features 96C. As indicated, with the stopper 24 compressed (e.g., as would be the case in assembly in a pre-filled syringe example), upon insertion of the plunger rod 22 into the stopper 24, the enlarged segment 94C engages with the capture portion 80G. This engagement couples the stopper 24 to the head portion 46C, but requires lower axial insertion forces Fi in order to insert the head portion 46C into the pocket 70G than a break loose force between the stopper 24 and the barrel 20, while still requiring a substantial, non-zero separation force in order to de-couple the stopper 24 from the head portion 46C.

It will be understood that the various head portion designs and features described above can be combined and/or utilized with any of the various pocket designs described above.

With the foregoing description taken into consideration, a method of assembling a plunger rod to a stopper can be described as follows.

A method of coupling a plunger rod to a stopper that has been positioned in a barrel of a syringe includes axially inserting a head portion of the plunger rod into a pocket of the stopper with an insertion force that is less than a break loose force defined between the stopper and the barrel of the syringe. Upon axially inserting the head portion of the plunger rod into a capture portion of the stopper the plunger rod is coupled to the stopper.

Inserting the head portion into the pocket can include sliding a tapered crown of the head portion of the plunger rod past one or more coupling projections corresponding to the coupling portion of the pocket in order to couple the plunger rod to the stopper. The one or more coupling projections include one or more longitudinally extending ribs and/or one or more circumferentially extending ribs. Each of the one or more coupling projections can have a leading edge and a trailing edge, where at least the leading edge is at least one of angled and rounded, and inserting the head portion into the pocket includes sliding the head portion longitudinally past the leading edge and/or the trailing edge of the one or more coupling projections. As previously referenced, the break loose force can be from 2N to 20N, for example. The stopper and the plunger rod also require application of a separation force in a longitudinal direction to decouple the plunger rod from the stopper once the plunger rod is inserted into the stopper. In various examples, the separation force is greater than the break loose force.

Inserting the head portion into the pocket can also include sliding an enlarged segment of the head portion of the plunger rod into one or more coupling recesses corresponding to the coupling portion of the pocket in order to couple the plunger rod to the stopper. The one or more coupling recesses may include one or more longitudinally-extending recesses and/or one or more circumferentially-extending recesses. Each of the one or more coupling recesses can have a leading edge and a trailing edge, where at least the leading edge is at least one of angled and rounded, and, inserting the head portion into the pocket includes sliding the head portion longitudinally past the leading edge of the one or more coupling recesses to seat an enlarged segment of the head portion (e.g., one or more retaining features) into the one or more coupling recesses. Again, and as previously referenced, the break loose force can be from 2N to 20N, for example. The stopper and the plunger rod also require application of a separation force in a longitudinal direction to decouple the plunger rod from the stopper once the plunger rod is inserted into the stopper. And, once again, in various examples, the separation force is greater than the break loose force.

### Examples of Potential Syringe Barrel Contents

The syringes of the present disclosure may be used in combination with different therapeutic compounds including, but not limited to, drugs and biologics such as Coagulation Factors, Cytokines, Epigenetic protein families, Growth Factors, Hormones, Peptides, Signal Transduction molecules, and mutations thereof; also including Amino Acids, Vaccines and/or combinations thereof. Therapeutic compounds further include antibodies, antisense, RNA interference made to the above biologics and their target receptors and mutations of thereof. Additional therapeutic compounds include Gene Therapy, Primary and Embryonic Stem Cells. Also included in the therapeutic compounds are antibodies, antisense, RNA interference to Protein Kinases, Esterases, Phosphatases, Ion channels, Proteases, structural proteins, membrane transport proteins, nuclear hormone receptors and/or combinations thereof. Additionally, it is to be understood that at least one of the therapeutic compounds identified herein used in the instant disclosure, also two or more therapeutic compounds listed in this application are considered to be within the purview of the present disclosure.

Examples of Coagulation Factors include, but are not limited to: Fibrinogen, Prothrombin, Factor I, Factor V, Factor X, Factor VII, Factor VIII, Factor XI, Factor XIII, Protein C, Platelets, Thromboplastin, and Co-factor of VIIa.

Examples of Cytokines include, but are not limited to: Lymphokines, Interleukins, Chemokines, Monokines, Interferons, and Colony stimulating factors.

Examples of Epigenetic protein families include, but are not limited to: ATPase family AAA domain-containing protein 2 (ATAD2A), ATPase family-AAA domain containing 2B (ATAD2B), ATPase family AAA domain containing-2B (ATAD2B), bromodomain adjacent to zinc finger domain-1A (BAZ1A), bromodomain adjacent to zinc finger domain-1B (BAZ1B), bromodomain adjacent to zinc finger domain-2A (BAZ2A), bromodomain adjacent to zinc finger domain-2A (BAZ2A), bromodomain adjacent to zinc finger domain-2B (BAZ2B), bromodomain-containing protein 1 (BRD1), Bromodomain containing protein 2-1st bromodomain (BRD2), Bromodomain containing protein 2-1st & 2nd bromodomains (BRD2), bromodomain-containing protein 2 isoform 1-bromodomain 2 (BRD2(2)), bromodomain-containing protein 3-bromodomain 1 (BRD3(1)), Bromodomain-containing protein 3-1st bromodomain (BRD3), Bromodomain-containing protein 3-1st & 2nd bromodomains (BRD3), bromodomain-containing protein 3-bromodomain 2 (BRD3(2)), Bromodomain containing protein 4-1st bromodomain (BRD4), bromodomain-containing protein 4 isoform long-bromodomains 1 and 2 (BRD4(1-2)), bromodomain-containing protein 4 isoform long-bromodomain 2 (BRD4(2)), bromodomain-containing protein 4 isoform short (BRD4(full-length-short-iso.)), Bromodomain containing protein 7 (BRD7), bromodomain containing 8-bromodomain 1 (BRD8(1)), bromodomain containing 8-bromodomain 2 (BRD8(2)), bromodomain-containing protein 9 isoform 1 (BRD9), Bromodomain containing testis-specific-1st bromodomain (BRDT), Bromodomain containing testis-specific-1st & 2nd bromodomains (BRDT), bromodomain testis-specific protein isoform b-bromodomain 2 (BRDT(2)), bromodomain and PHD finger containing-1 (BRPF1), bromodomain and PHD finger containing-3 (BRPF3), bromodomain and PHD finger containing-3 (BRPF3), Bromodomain and WD repeat-containing 3-2nd bromodomain (BRWD3(2)), Cat eye syndrome critical region protein 2 (CECR2), CREB binding protein (CREBBP), E1A binding protein p300 (EP300), EP300 (EP300), nucleosome-remodeling factor subunit BPTF isoform 1 (FALZ), Nucleosome-remodeling factor subunit BPT (FALZ), Euchromatic histone-lysine N-methyltransferase 2 (EHMT2), Histone Acetyltransferase-KAT2A (GCN5L2), Euchromatic histone-lysine N-methyltransferase 1 (EHMT1), Histone-lysine N-methyltransferase MLL (MLL), Polybromo 1-1st bromodomain (PB1(1)), Polybromo 1-2nd bromodomain (PB1(2)), polybromo 1-bromodomain 2 (PBRM1(2)), polybromo 1-bromodomain 5 (PBRM1(5)), Histone acetyltransferase KAT2B (PCAF), PH-interacting protein-1st bromodomain (PHIP(1)), PH-interacting protein-2nd bromodomain (PHIP(2)), Protein kinase C-binding protein 1 (PRKCBP1), Protein arginine N-methyltransferase 3 (PRMT3), SWI/SNF related-matrix associated-actin dependent regulator of chromatin-subfamily a-member 2 (SMARCA2), SWI/SNF related-matrix associated-actin dependent regulator of chromatin-subfamily a-member 4 (SMARCA4), Nuclear body protein-SP110 (SP110), Nuclear body protein—SP140 (SP140), Transcription initiation factor TFIID subunit 1 (TAF1(1-2)), TAF1 RNA polymerase II-TATA box binding protein (TBP)-associated factor-250 kDa-bromodomain 2 (TAF1(2)), Transcription initiation factor TFIID subunit 1-like-1st bromodomain (TAF1L(1)), Transcription initiation factor TFIID subunit 1-like-2nd bromodomain (TAF1L(2)), tripartite motif containing 24 (TRIM24(Bromo.)), tripartite motif containing 24 (TRIM24(PHD-Bromo.)), E3 ubiquitin-protein ligase TRIM33 (TRIM33), tripartite motif containing 33 (TRIM33(PHD-Bromo.)), WD repeat 9-1st bromodomain (WDR9(1)), and WD repeat 9-2nd bromodomain (WDR9(2)).

Examples of growth factors include, but are not limited to: nerve growth factor (NGF), vascular endothelial growth factor (VEGF), platelet-derived growth factor (PDGF), C-fos-induced growth factor (FIGF), platelet-activating factor (PAF), transforming growth factor beta (TGF-β), bone morphogenetic proteins (BMPs), Activin, inhibin, fibroblast growth factors (FGFs), granulocyte-colony stimulating factor (G-CSF), granulocyte-macrophage colony stimulating factor (GM-CSF), glial cell line-derived neurotrophic factor (GDNF), growth differentiation factor-9 (GDF9), epidermal growth factor (EGF), transforming growth factor-α (TGF-α), growth factor (KGF), migration-stimulating factor (MSF), hepatocyte growth factor-like protein (HGFLP), hepatocyte growth factor (HGF), hepatoma-derived growth factor (HDGF), and Insulin-like growth factors.

Examples of Hormones include, but are not limited to: Amino acid derived (such as melatonin and thyroxine), Thyrotropin-releasing hormone, Vasopressin, Insulin, Growth Hormones, Glycoprotein Hormones, Luteinizing Hormone, Follicle-stimulating Hormone, Thyroid-stimulating hormone, Eicosanoids, Arachidonic acid, Lipoxins, Prostaglandins, Steroid, Estrogens, Testosterone, Cortisol, and Progestogens.

Examples of Proteins and Peptides and Signal Transduction molecules include, but are not limited to: Ataxia Telangiectasia Mutated, Tumor Protein p53, Checkpoint kinase 2, breast cancer susceptibility protein, Double-strand break repair protein, DNA repair protein RAD50, Nibrin, p53-binding protein, Mediator of DNA damage checkpoint protein, H2A histone family member X, Microcephalin, C-terminal-binding protein 1, Structural maintenance of chromosomes protein 1A, Cell division cycle 25 homolog A (CDC25A), forkhead box O3 (forkhead box O3), nuclear factor of kappa light polypeptide gene enhancer in B-cells inhibitor, alpha (NFKBIA), nuclear factor (erythroid-derived 2)-like 2 (NFE2L2), Natriuretic peptide receptor A (NPR1), Tumor necrosis factor receptor superfamily, member 11a (TNFRSF11A), v-rel reticuloendotheliosis viral oncogene homolog A (avian) (RELA), Sterol regulatory element binding transcription factor 2 (SREBF2), CREB regulated transcription coactivator 1 (CRTC1), CREB regulated transcription coactivator 2 (CRTC2), X-box binding protein 1 (XBP1), and Catenin beta 1 (cadherin-associated protein or CTNNB1).

Examples of G Protein-Coupled Receptors (GPCR) include, but are not limited to: Adenosine receptor family, Adrenergic receptor family, Angiotensin II receptor, Apelin receptor, Vasopressin receptor family, Brain-specific angiogenesis inhibitor family, Bradykinin receptor family, Bombesin receptor family, Complement component 3a receptor 1, Complement component 5a receptor 1, Calcitonin receptor family, Calcitonin receptor-like family, Calcium-sensing receptor, Cholecystokinin A receptor (CCK1), Cholecystokinin B receptor (CCK2), Chemokine (C-C motif) receptor family, Sphingosine 1-phosphate receptor family, Succinic receptor, Cholinergic receptor family. Chemokine-like receptor family, Cannabinoid receptor family, Corticotropin releasing hormone receptor family, prostaglandin D2 receptor, Chemokine C-X3-C receptor family, Chemokine (C-X-C motif) receptor family, Burkitt lymphoma receptor, Chemokine (C-X-C motif) receptor family, Cysteinyl leukotriene receptor 2 (CYSLT2), chemokine receptor (FY), Dopamine receptor family, G protein-coupled receptor 183 (GPR183), Lysophosphatidic acid receptor family, Endothelin receptor family, Coagulation factor II (thrombin) receptor family, Free fatty acid receptor family, Formylpeptide receptor family, Follicle stimulating hormone receptor (FSHR), gamma-aminobutyric acid (GABA) B receptor, Galanin receptor family, Glucagon receptor, Growth hormone releasing hormone receptor (GHRH), Ghrelin receptor (ghrelin), Growth hormone secretagogue receptor 1b (GHSR1b), Gastric inhibitory polypeptide receptor (GIP), Glucagon-like peptide receptor family, Gonadotropin-releasing hormone receptor (GnRH), pyroglutamylated RFamide peptide receptor (QRFPR), G protein-coupled bile acid receptor 1 (GPBA), Hydroxycarboxylic acid receptor family, Lysophosphatidic acid receptor 4 (LPA4) Lysophosphatidic acid receptor 5 (GPR92), G protein-coupled receptor 79 pseudogene (GPR79), Hydroxycarboxylic acid receptor 1 (HCA1), G-protein coupled receptor (C5L2, FFA4, FFA4, FFA4, GPER, GPR1, GPR101, GPR107, GPR119, GPR12, GPR123, GPR132, GPR135, GPR139, GPR141, GPR142, GPR143, GPR146, GPR148, GPR149, GPR15, GPR150, GPR151, GPR152, GPR157, GPR161, GPR162, GPR17, GPR171, GPR173, GPR176, GPR18, GPR182, GPR20, GPR22, GPR25, GPR26, GPR27, GPR3, GPR31, GPR32, GPR35, GPR37L1, GPR39, GPR4, GPR45, GPR50, GPR52, GPR55, GPR6, GPR61, GPR65, GPR75, GPR78, GPR83, GPR84, GPR85, GPR88, GPR97, TM7SF1), Metabotropic glutamate receptor family, Gastrin releasing peptide receptor (BB2), Orexin receptor family, Histamine receptor family, 5-hydroxytryptamine receptor family, KISS1-derived peptide receptor (kisspeptin), Leucine-rich repeat-containing G protein-coupled receptor family, choriogonadotropin receptor (LH), Leukotriene B4 receptor (BLT1), Adenylate Cyclase Activating Polypeptide 1 Receptor 1 (mPAC1), Motilin receptor, Melanocortin receptor family, Melanin concentrating hormone receptor 1 (MCH1), Neuropeptide Y1 receptor (Y1), Neuropeptide Y2 receptor (NPY2R), Opioid receptor family, Oxytocin receptor (OT), P2Y Purinoceptor 12 (mP2Y12), P2Y Purinoceptor 6 (P2Y6), Pancreatic polypeptide receptor family, Platelet-activating factor receptor family, Prostaglandin E receptor family, Prostanoid IP1 receptor (IP1), MAS-related GPR, member family, Rhodopsin (Rhodopsin), Relaxin family peptide receptor family, Somatostatin receptor family, Tachykinin receptor family, Melatonin receptor family, Urotensin receptor family, Vasoactive intestinal peptide receptor 1 (mVPAC1), Neuromedin B Receptor (BB1), Neuromedin U receptor 1 (NMU1), Neuropeptides B/W receptor family, Neuropeptide FF receptor 1 (NPFF1), neuropeptide S receptor 1 (NPS receptor), Neuropeptide Y receptor family, Neurotensin receptor 1 (NTS1), Opsin 5 (OPN5), Opioid receptor-like receptor (NOP), Oxoeicosanoid (OXE) receptor 1 (OXE), Oxoglutarate (alpha-ketoglutarate) receptor 1 (OXGR1), Purinergic receptor family, Pyrimidinergic receptor family, Prolactin releasing hormone receptor (PRRP), Prokineticin receptor family, Platelet activating receptor (PAF), Prostaglandin F receptor family, Prostaglandin 12 (prostacyclin) receptor family, Parathyroid hormone receptor family, muscarinic acetylcholine receptors (such as rM4), Prostanoid DP2 receptor (rGPR44), Prokineticin receptor family, Relaxin family peptide receptor family, Secretin receptor (secretin), Frizzled class receptor (Smoothened), trace amine associated receptor family, Tachykinin family, Thromboxane A2 receptor (TP), Thyrotropin-releasing hormone receptor (TRH1), and Thyroid Stimulating Hormone Receptor (TSH).

Examples of nuclear hormone receptors include, but are not limited to: Androgen receptor (AR), Estrogen related receptor alpha (ESRRA), Estrogen receptor 1 (ESR1), Nuclear receptor subfamily 1-group H—member 4 (NR1H4), Nuclear receptor subfamily 3-group C—member 1 (glucocorticoid receptor) (NR3C1), Nuclear receptor subfamily 1-group H—member 3 (Liver X receptor α) (NR1H3), Nuclear receptor subfamily 1-group H—member 2 (Liver X receptor β) (NR1H2), Nuclear receptor subfamily 1-group H—member 2 (Liver X receptor β) (NR1H2), Nuclear receptor subfamily 3-group C—member 2 (Mineralocorticoid receptor) (NR3C2), Peroxisome Proliferator Activated Receptor alpha (PPARA), Peroxisome Proliferator Activated Receptor gamma (PPARG), Peroxisome Proliferator Activated Receptor delta (PPARD), Progesterone receptor α (PGR), Progesterone receptor β (PGR), Retinoic acid receptor-alpha (RARA), Retinoic acid receptor-beta (RARB), Retinoid X receptor-alpha (RXRA), Retinoid X receptor-gamma (RXRG), Thyroid hormone receptor-alpha (THRA), Thyroid hormone receptor-beta (THRB), Retinoic acid-related orphan receptor, Liver X receptor, Farnesoid X receptor, Vitamin D receptor, Pregnane X receptor, Constitutive androstane receptor, Hepatocyte nuclear factor 4, Oestrogen receptor, Oestrogen-related receptor, Glucocortioic receptor, and Nerve growth factor-induced-B, Germ cell nuclear factor.

Examples of membrane transport proteins include, but are not limited to: ATP-binding cassette (ABC) superfamily, solute carrier (SLC) superfamily, multidrug resistance protein 1 (P-glycoprotein), organic anion transporter 1, and proteins such as EAAT3, EAAC1, EAAT1, GLUT1, GLUT2, GLUT9, GLUT10, rBAT, AE1, NBC1, KNBC, CHED2, BTR1, NABC1, CDPD, SGLT1, SGLT2, NIS, CHT1, NET, DAT, GLYT2, CRTR, BOAT1, SIT1, XT3, y+LAT1, BAT1, NHERF1, NHE6, ASBT, DMT1, DCT1, NRAMP2, NKCC2, NCC, KCC3, NACT, MCT1, MCT8, MCT12, SLD, VGLUT3, THTR1, THTR2, PIT2, GLVR2, OCTN2, URAT1, NCKX1, NCKX5, CIC, PiC, ANTI, ORNT1, AGC1, ARALAR, Citrin, STLN2, aralar2, TPC, MUP1, MCPHA, CACT, GC1, PHC, DTD, CLD, DRA, PDS, Prestin, TAT1, FATP4, ENT3, ZnT2, ZnT10, AT1, NPT2A, NPT2B, HHRH, CST, CDG2F, UGAT, UGTL, UGALT, UGT1, UGT2, FUCT1, CDG2C, NST, PAT2, G6PT1, SPX4, ZIP4, LIV4, ZIP13, LZT-Hs9, FPN1, MTP1, IREG1, RHAG, AIM1, PCFT, FLVCR1, FLVCR2, RFT1, RFT2, RFT3, OATP1B1, OATP1B3, and OATP2A1.

Examples of structural proteins include, but are not limited to: tubulin, heat shock protein, Microtubule-stabilizing proteins, Oncoprotein 18, stathmin, kinesin-8 and kinesin-14 family, Kip3, and Kif18A.

Examples of proteases include, but are not limited to ADAM (a disintegrin and metalloprotease) family.

Examples of Protein kinases include, but are not limited to: AP2 associated kinase, Homo sapiens ABL proto-oncogene 1-non-receptor tyrosine-protein kinase family, c-abl oncogene 1 receptor tyrosine kinase family, v-abl Abelson murine leukemia viral oncogene homolog 2, activin A receptor family, chaperone-ABC1 activity of bc1 complex homolog (S. pombe) (ADCK3), aarF domain containing kinase 4 (ADCK4), v-akt murine thymoma viral oncogene homolog family, anaplastic lymphoma receptor tyrosine kinase family, protein kinase A family, protein kinase B family, ankyrin repeat and kinase domain containing 1 (ANKK1), NUAK family-SNF1-like kinase, mitogen-activated protein kinase kinase kinase family aurora kinase A (AURKA), aurora kinase B (AURKB), aurora kinase C (AURKC), AXL receptor tyrosine kinase (AXL), BMP2 inducible kinase (BIKE), B lymphoid tyrosine kinase (BLK), bone morphogenetic protein receptor family, BMX non-receptor tyrosine kinase (BMX), v-raf murine sarcoma viral oncogene homolog B1 (BRAF), protein tyrosine kinase 6 (BRK), BR serine/threonine kinase family, Bruton agammaglobulinemia tyrosine kinase (BTK), calcium/calmodulin-dependent protein kinase family, cyclin-dependent kinase family, cyclin-dependent kinase-like family, CHK1 checkpoint homolog (S. pombe) (CHEK1), CHK2 checkpoint homolog (S. pombe) (CHEK2), Insulin receptor, isoform A (INSR), Insulin receptor, isoform B (INSR), rho-interacting serine/threonine kinase (CIT), v-kit Hardy-Zuckerman 4 feline sarcoma viral oncogene homolog (KIT), CDC-Like Kinase family-Hepatocyte growth factor receptor (MET), Proto-oncogene tyrosine-protein kinase receptor, colony-stimulating factor family receptor, c-src tyrosine kinase (CSK), casein kinase family, megakaryocyte-associated tyrosine kinase (CTK), death-associated protein kinase family, doublecortin-like kinase family, discoidin domain receptor tyrosine kinase, dystrophia myotonica-protein kinase (DMPK), dual-specificity tyrosine-(Y)-phosphorylation regulated kinase family, epidermal growth factor receptor family, eukaryotic translation initiation factor 2-alpha kinase 1 (EIF2AK1), EPH receptor family, Ephrin type-A receptor family, Ephrin type-B receptor family, v-erb-b2 erythroblastic leukemia viral oncogene homolog family, mitogen-activated protein kinase family, endoplasmic reticulum to nucleus signaling 1 (ERN1), PTK2 protein tyrosine kinase 2 (FAK), fer (fps/fes related) tyrosine kinase (FER). feline sarcoma oncogene (FES), Fibroblast growth factor receptor family, Gardner-Rasheed feline sarcoma viral (v-fgr) oncogene homolog (FGR), fms-related tyrosine kinase family, Fms-related tyrosine kinase family, fyn-related kinase (FRK), FYN oncogene related to SRC, cyclin G associated kinase (GAK), eukaryotic translation initiation factor 2 alpha kinase, Growth hormone receptor. G protein-coupled receptor kinase 1 (GRK1), G protein-coupled receptor kinase family, glycogen synthase kinase family, germ cell associated 2 (haspin) (HASPIN), Hemopoietic cell kinase (HCK), homeodomain interacting protein kinase family, mitogen-activated protein kinase kinase kinase kinase family, hormonally up-regulated Neu-associated kinase (HUNK), intestinal cell (MAK-like) kinase (ICK), Insulin-like growth factor 1 receptor (IGF1R), conserved helix-loop-helix ubiquitous kinase (IKK-alpha), inhibitor of kappa light polypeptide gene enhancer in B-cells-kinase beta family, insulin receptor (INSR), insulin receptor-related receptor (INSRR), interleukin-1 receptor-associated kinase family, IL2-inducible T-cell kinase (ITK), Janus kinase family, Kinase Insert Domain Receptor, v-kit Hardy-Zuckerman 4 feline sarcoma viral oncogene homolog, lymphocyte-specific protein tyrosine kinase (LCK), LIM domain kinase family, serine/threonine kinase family leucine-rich repeat kinase family, v-yes-1 Yamaguchi sarcoma viral related oncogene homolog (LYN), male germ cell-associated kinase (MAK); MAP/microtubule affinity-regulating kinase family such as microtubule associated serine/threonine kinase family, maternal embryonic leucine zipper kinase, c-mer proto-oncogene tyrosine kinase (MERTK), met proto-oncogene (hepatocyte growth factor receptor), MAP kinase interacting serine/threonine kinase family, myosin light chain kinase family, mixed lineage kinase domain-like protein isoform, CDC42 binding protein kinase family, serine/threonine kinase family, macrophage stimulating 1 receptor (c-met-related tyrosine kinase) (MST1R), mechanistic target of rapamycin (serine/threonine kinase) (MTOR), muscle-skeletal-receptor tyrosine kinase (MUSK), myosin light chain kinase family, NIMA (never in mitosis gene a)-related kinase family, serine/threonine-protein kinase NIM1 (NIM1), nemo-like kinase (NLK), oxidative-stress responsive 1 (OSR1), p21 protein (Cdc42/Rac)-activated kinase family, PAS domain containing serine/threonine kinase, Platelet-derived growth factor receptor family, 3-phosphoinositide dependent protein kinase-1 (PDPK1), Calcium-dependent protein kinase 1, phosphorylase kinase gamma family, Phosphatidylinositol 4,5-bisphosphate 3-kinase, phosphoinositide-3-kinase family, phosphatidylinositol 4-kinase family. phosphoinositide kinase, FYVE finger containing, Pim-1 oncogene (PIM1), pim-2 oncogene (PIM2), pim-3 oncogene (PIM3), phosphatidylinositol-4-phosphate 5-kinase family, phosphatidylinositol-5-phosphate 4-kinase family protein kinase, membrane associated tyrosine/threonine 1 (PKMYT1), protein kinase N family, polo-like kinase family, protein kinase C family, protein kinase D family, cGMP-dependent protein kinase family, eukaryotic translation initiation factor 2-alpha kinase 2 (PRKR), X-linked protein kinase (PRKX), Prolactin receptor (PRLR), PRP4 pre-mRNA processing factor 4 homolog B (yeast) (PRP4), PTK2B protein tyrosine kinase 2 beta (PTK2B), SIK family kinase 3 (QSK), v-raf-1 murine leukemia viral oncogene homolog 1 (RAF1), Neurotrophic tyrosine kinase receptor type family, receptor (TNFRSF)-interacting serine-threonine kinase family, dual serine/threonine and tyrosine protein kinase (RIPK5), Rho-associated, coiled-coil containing protein kinase family, c-ros oncogene 1, receptor tyrosine kinase (ROS1), ribosomal protein S6 kinase family, SH3-binding domain kinase 1 (SBK1), serum/glucocorticoid regulated kinase family, Putative uncharacterized serine/threonine-protein kinase (Sugen kinase 110) (SgK110), salt-inducible kinase family, SNF related kinase (SNRK), src-related kinase, SFRS protein kinase family; Spleen tyrosine kinase (SYK) such as TAO kinase family; TANK-binding kinase 1 (TBK1) such as tec protein tyrosine kinase (TEC), testis-specific kinase 1 (TESK1), transforming growth factor, beta receptor family, tyrosine kinase with immunoglobulin-like and EGF-like domains 1 (TIE1), TEK tyrosine kinase, endothelial (TIE2), Angiopoietin-1 receptor (Tie2), tousled-like kinase family, TRAF2 and NCK interacting kinase (TN IK), non-receptor tyrosine kinase family, TNNI3 interacting kinase (TNNI3K), transient receptor potential cation channel, testis-specific serine kinase family, TTK protein kinase (TTK), TXK tyrosine kinase (TXK), Tyrosine kinase 2 (TYK2), TYROS protein tyrosine kinase (TYROS), unc-51-like kinase family, phosphatidylinositol 3-kinase, vaccinia related kinase 2 (VRK2), WEE1 homolog family, WNK lysine deficient protein kinase family, v-yes-1 Yamaguchi sarcoma viral oncogene homolog 1 (YES), sterile alpha motif and leucine zipper containing kinase AZK (ZAK), and zeta-chain (TCR) associated protein kinase 70 kDa (ZAP70).

Cell therapy using cells that are derived primarily from: endoderm such as Exocrine secretory epithelial cells and Hormone-secreting cells; ectoderm such as Keratinizing epithelial cells, Wet stratified barrier epithelial cells, Sensory transducer cells, Autonomic neuron cells, Sense organ and peripheral neuron supporting cells, Central nervous system neurons and glial cells, Lens cells; mesoderm such as Metabolism and storage cells, Barrier function cells (lung, gut, exocrine glands and urogenital tract), Extracellular matrix cells, Contractile cells, Blood and immune system cells, Germ cells, Nurse cell, Interstitial cells and combinations thereof. Additionally, in the scope of the invention are cells that are genetically, chemically or physically altered or otherwise modified.

Examples of Exocrine secretory epithelial cells include but are not limited to: Salivary gland mucous cell, Salivary gland number 1, Von Ebner's gland cell in tongue, Mammary gland cell, Lacrimal gland cell, Ceruminous gland cell in ear, Eccrine sweat gland dark cell, Eccrine sweat gland clear cell, Apocrine sweat gland cell, Gland of Moll cell in eyelid, Sebaceous gland cell, Bowman's gland cell in nose, Brunner's gland cell in duodenum, Seminal vesicle cell, Prostate gland cell, Bulbourethral gland cell, Bartholin's gland cell, Gland of Littre cell, Uterus endometrium cell, Isolated goblet cell of respiratory and digestive tracts, Stomach lining mucous cell, Gastric gland zymogenic cell, Gastric gland oxyntic cell, Pancreatic acinar cell, Paneth cell of small intestine, Type II pneumocyte of lung, and Clara cell of lung; Hormone-secreting cells including, but not limited to: Anterior pituitary cells, Intermediate pituitary cell, Magnocellular neurosecretory cells, Gut and respiratory tract cells, Thyroid gland cells, Parathyroid gland cells, Adrenal gland cells, Leydig cell of testes secreting testosterone, Theca interna cell of ovarian follicle secreting estrogen, Corpus luteum cell of ruptured ovarian follicle secreting progesterone, Juxtaglomerular cell, Macula densa cell of kidney, Peripolar cell of kidney, Mesangial cell of kidney, and Pancreatic islets; Keratinizing epithelial cells including, but not limited to: Epidermal keratinocyte, Epidermal basal cell, Keratinocyte of fingernails and toenails, Nail bed basal cell, Medullary hair shaft cell, Cortical hair shaft cell, Cuticular hair shaft cell, Cuticular hair root sheath cell, Hair root sheath cell of Huxley's layer, Hair root sheath cell of Henle's layer, External hair root sheath cell, and Hair matrix cell; Wet stratified barrier epithelial cells including, but not limited to: Surface epithelial cell of stratified squamous epithelium and basal cell of epithelia of cornea, tongue, oral cavity, esophagus, anal canal, distal urethra and vagina, and Urinary epithelium cell; Sensory transducer cells including, but not limited to: Auditory inner hair cell of organ of Corti, Auditory outer hair cell of organ of Corti, Basal cell of olfactory epithelium, Cold-sensitive primary sensory neurons, Heat-sensitive primary sensory neurons, Merkel cell of epidermis, Olfactory receptor neuron, Pain-sensitive primary sensory neurons, Photoreceptor cells of retina in eye, Proprioceptive primary sensory neurons, Touch-sensitive primary sensory neurons, Type I carotid body cell, Type II carotid body cell, Type I hair cell of vestibular system of ear, Type II hair cell of vestibular system of ear, and Type I taste bud cell; Autonomic neuron cells including, but not limited to: Cholinergic neural cell, Adrenergic neural cell, and Peptidergic neural cell; Sense organ and peripheral neuron supporting cells including, but not limited to: Inner pillar cell of organ of Corti, Outer pillar cell of organ of Corti, Inner phalangeal cell of organ of Corti, Outer phalangeal cell of organ of Corti, Border cell of organ of Corti, Hensen cell of organ of Corti, Vestibular apparatus supporting cell, Taste bud supporting cell, Olfactory epithelium supporting cell, Schwann cell, Satellite glial cell, and Enteric glial cell; Central nervous system neurons and glial cells including, but not limited to: Astrocyte, Neuron cells, Oligodendrocyte, and Spindle neuron; Lens cells including, but not limited to: Anterior lens epithelial cell, and Crystallin-containing lens fiber cell; Metabolism and storage cells including, but not limited to: Adipocytes, and Liver lipocyte; Barrier function cells including, but not limited to: Kidney parietal cell, Kidney glomerulus podocyte, Kidney proximal tubule brush border cell, Loop of Henle thin segment cell, Kidney distal tubule cell, Kidney collecting duct cell, Principal cells, Intercalated cells, Type I pneumocyte, Pancreatic duct cell, Nonstriated duct cell, Principal cell, Intercalated cell, Duct cell, Intestinal brush border cell, Exocrine gland striated duct cell, Gall bladder epithelial cell, Ductulus efferens nonciliated cell, Epididymal principal cell, and Epididymal basal cell; Extracellular matrix cells including, but not limited to: Ameloblast epithelial cell, Planum semilunatum epithelial cell of vestibular system of ear, Organ of Corti interdental epithelial cell, Loose connective tissue fibroblasts, Corneal fibroblasts, Tendon fibroblasts, Bone marrow reticular tissue fibroblasts, Other nonepithelial fibroblasts, Pericyte, Nucleus pulposus cell of intervertebral disc, Cementoblast/cementocyte, Odontoblast/odontocyte, Hyaline cartilage chondrocyte, Fibrocartilage chondrocyte, Elastic cartilage chondrocyte, Osteoblast/osteocyte, Osteoprogenitor cell, Hyalocyte of vitreous body of eye, Stellate cell of perilymphatic space of ear, Hepatic stellate cell, and Pancreatic stelle cell; Contractile cells including, but not limited to: Skeletal muscle cell, Satellite cell, Heart muscle cells, Smooth muscle cell, Myoepithelial cell of iris, and Myoepithelial cell of exocrine glands; Blood and immune system cells including, but not limited to: Erythrocyte, Megakaryocyte, Monocyte, Connective tissue macrophage, Epidermal Langerhans cell, Osteoclast, Dendritic cell, Microglial cell, Neutrophil granulocyte, Eosinophil granulocyte, Basophil granulocyte, Hybridoma cell, Mast cell, Helper T cell, Suppressor T cell, Cytotoxic T cell, Natural Killer T cell, B cell, Natural killer cell, Reticulocyte, Stem cells, and committed progenitors for the blood and immune system; Germ cells including, but not limited to: Oogonium/Oocyte, Spermatid, Spermatocyte, Spermatogonium cell, and Spermatozoon; Nurse cell including, but not limited to: Ovarian follicle cell, and Sertoli cell, Thymus epithelial cell; Interstitial cells including, but not limited to: Interstitial kidney cells and any combination of the foregoing.

Non-limiting examples of other known biologics include, but are not limited to: Abbosynagis, Abegrin, Actemra, AFP-Cide, Antova, Arzerra, Aurexis, Avastin, Benlysta, Bexxar, Blontress, Bosatria, Campath, CEA-Cide, CEA-Scan, Cimzia, Cyramza, Ektomab, Erbitux, FibriScint, Gazyva, Herceptin, hPAM4-Cide, HumaSPECT, HuMax-CD4, HuMax-EGFr, Humira, HuZAF, Hybri-ceaker, Ilaris, Indimacis-125, Kadcyla, Lemtrada, LeukArrest, LeukoScan, Lucentis, Lymphomun, LymphoScan, LymphoStat-B, MabThera, Mycograb, Mylotarg, Myoscint, NeutroSpec, Numax, Nuvion, Omnitarg, Opdivo, Orthoclone OKT3, OvaRex, Panorex, Prolia, Prostascint, Raptiva, Remicade, Removab, Rencarex, ReoPro, Rexomun, Rituxan, RoActemra, Scintimun, Simponi, Simulect, Soliris, Stelara, Synagis, Tactress, Theracim, Theragyn, Theraloc, Tysabri, Vectibix, Verluma, Xolair, Yervoy, Zenapax, and Zevalin and combinations thereof.

Non-limiting examples of known Monoclonal antibodies include, but are not limited to: 3F8, 8H9, Abagovomab, Abciximab, Abituzumab, Abrilumab, Actoxumab, Adalimumab, Adecatumumab, Aducanumab, Afasevikumab, Afelimomab, Afutuzumab, Alacizumab pegol, ALD518, ALD403, Alemtuzumab, Alirocumab, Altumomab pentetate, Amatuximab, AMG 334, Anatumomab mafenatox, Anetumab ravtansine, Anifrolumab, Anrukinzumab, Apolizumab, Arcitumomab, Ascrinvacumab, Aselizumab, Atezolizumab, Atinumab, Atlizumab, Atorolimumab, Avelumab, Bapineuzumab, Basiliximab, Bavituximab, Bectumomab, Begelomab, Belimumab, Benralizumab, Bertilimumab, Besilesomab, Bevacizumab, Bezlotoxumab, Biciromab, Bimagrumab, Bimekizumab, Bivatuzumab mertansine, Bleselumab, Blinatumomab, Blontuvetmab, Blosozumab, Bococizumab, Brazikumab, Brentuximab vedotin, Briakinumab, Brodalumab, Brolucizumab, Brontictuzumab, Burosumab, Cabiralizumab, Canakinumab, Cantuzumab mertansine, Cantuzumab ravtansine, Caplacizumab, Capromab pendetide, Carlumab, Carotuximab, Catumaxomab, cBR96-doxorubicin immunoconjugate, Cedelizumab, Cergutuzumab amunaleukin, Certolizumab pegol, Cetuximab, Citatuzumab bogatox, Cixutumumab, Clazakizumab, Clenoliximab, Clivatuzumab tetraxetan, Codrituzumab, Coltuximab ravtansine, Conatumumab, Concizumab, CR6261, Crenezumab, Crotedumab, Dacetuzumab, Daclizumab, Dalotuzumab, Dapirolizumab pegol, Daratumumab, Dectrekumab, Demcizumab, Denintuzumab mafodotin, Denosumab, Depatuxizumab mafodotin, Derlotuximab biotin, Detumomab, Dinutuximab, Diridavumab, Domagrozumab, Dorlimomab aritox, Drozitumab, Duligotumab, Dupilumab, Durvalumab, Dusigitumab, Ecromeximab, Eculizumab, Edobacomab, Edrecolomab, Efalizumab, Efungumab, Eldelumab, Elgemtumab, Elotuzumab, Elsilimomab, Emactuzumab, Emibetuzumab, Emicizumab, Enavatuzumab, nfortumab vedotin, Enlimomab pegol, Enoblituzumab, Enokizumab, Enoticumab, Ensituximab, Epitumomab cituxetan, Epratuzumab, Erenumab, Erlizumab, Ertumaxomab, Etaracizumab, Etrolizumab, Evinacumab, Evolocumab, Exbivirumab, Fanolesomab, Faralimomab, Farletuzumab, Fasinumab, FBTA05, Felvizumab, Fezakinumab, Fibatuzumab, Ficlatuzumab, Figitumumab, Firivumab, Flanvotumab, Fletikumab, Fontolizumab, Foralumab, Foravirumab, Fresolimumab, Fulranumab, Futuximab, Galcanezumab, Galiximab, Ganitumab, Gantenerumab, Gavilimomab, Gemtuzumab ozogamicin, Gevokizumab, Girentuximab, Glembatumumab vedotin, Golimumab, Gomiliximab, Guselkumab, Ibalizumab, Ibritumomab tiuxetan, Icrucumab, Idarucizumab, Igovomab, IMA-638, IMAB362, Imalumab, Imciromab, Imgatuzumab, Inclacumab, Indatuximab ravtansine, Indusatumab vedotin, Inebilizumab, Infliximab, Inolimomab, Inotuzumab ozogamicin, Intetumumab, Ipilimumab, Iratumumab, Isatuximab, Itolizumab, Ixekizumab, Keliximab, Labetuzumab, Lambrolizumab, Lampalizumab, Lanadelumab, Landogrozumab, Laprituximab emtansine, LBR-101/PF0442g7429, Lebrikizumab, Lemalesomab, Lendalizumab, Lenzilumab, Lerdelimumab, Lexatumumab, Libivirumab, Lifastuzumab vedotin, Ligelizumab, Lilotomab satetraxetan, Lintuzumab, Lirilumab, Lodelcizumab, Lokivetmab, Lorvotuzumab mertansine, Lucatumumab, Lulizumab pegol, Lumiliximab, Lumretuzumab, LY2951742, Mapatumumab, Margetuximab, Maslimomab, Matuzumab, Mavrilimumab, Mepolizumab, Metelimumab, Milatuzumab, Minretumomab, Mirvetuximab soravtansine, Mitumomab, Mogamulizumab, Monalizumab, Morolimumab, Motavizumab, Moxetumomab pasudotox, Muromonab-CD3, Nacolomab tafenatox, Nam ilumab, Naptumomab estafenatox, Naratuximab emtansine, Narnatumab, Natalizumab, Navicixizumab, Navivumab, Nebacumab, Necitumumab, Nemolizumab, Nerelimomab, Nesvacumab, Nimotuzumab, Nivolumab, Nofetumomab merpentan, Obiltoxaximab, Obinutuzumab, Ocaratuzumab, Ocrelizumab, Odulimomab, Ofatumumab, Olaratumab, Olokizumab, Omalizumab, Onartuzumab, Ontuxizumab, Opicinumab, Oportuzumab monatox, Oregovomab, Orticumab, Otelixizumab, Otlertuzumab, Oxelumab, Ozanezumab, Ozoralizumab, Pagibaximab, Palivizumab, Pamrevlumab, Panitumumab, Pankomab, Panobacumab, Parsatuzumab, Pascolizumab, Pasotuxizumab, Pateclizumab, Patritumab, Pembrolizumab, Pemtumomab, Perakizumab, Pertuzumab, Pexelizumab, Pidilizumab, Pinatuzumab vedotin, Pintumomab, Placulumab, Plozalizumab, Pogalizumab, Polatuzumab vedotin, Ponezumab, Prezalizumab, Priliximab, Pritoxaximab, Pritumumab, PRO 140, Quilizumab, Racotumomab, Radretumab, Rafivirumab, Ralpancizumab, Ramucirumab, Ranibizumab, Raxibacumab, Refanezumab, Regavirumab, Reslizumab, Rilotumumab, Rinucumab, Risankizumab, Rituximab, Rivabazumab pegol, Robatumumab, Roledumab, Romosozumab, Rontalizumab, Rovalpituzumab tesirine, Rovelizumab, Ruplizumab, Sacituzumab govitecan, Samalizumab, Sapelizumab, Sarilumab, Satumomab pendetide, Secukinumab, Seribantumab, Setoxaximab, Sevirumab, SGN-CD19A, SGN-CD33A, Sibrotuzumab, Sifalimumab, Siltuximab, Simtuzumab, Siplizumab, Sirukumab, Sofituzumab vedotin, Solanezumab, Solitomab, Sonepcizumab, Sontuzumab, Stamulumab, Sulesomab, Suvizumab, Tabalumab, Tacatuzumab tetraxetan, Tadocizumab, Talizumab, Tamtuvetmab, Tanezumab, Taplitumomab paptox, Tarextumab, Tefibazumab, Telimomab aritox, Tenatumomab, Teneliximab, Teplizumab, Teprotumumab, Tesidolumab, Tetulomab, Tezepelumab, TGN1412, Ticilimumab, Tigatuzumab, Tildrakizumab, Timolumab, Tisotumab vedotin, TNX-650, Tocilizumab, Toralizumab, Tosatoxumab, Tositumomab, Tovetumab, Tralokinumab, Trastuzumab, Trastuzumab emtansine, TRBS07, Tregalizumab, Tremelimumab, Trevogrumab, Tucotuzumab celmoleukin, Tuvirumab, Ublituximab, Ulocuplumab, Urelumab, Urtoxazumab, Ustekinumab, Utomilumab, Vadastuximab talirine, Vandortuzumab vedotin, Vantictumab, Vanucizumab, Vapaliximab, Varlilumab, Vatelizumab, Vedolizumab, Veltuzumab, Vepalimomab, Vesencumab, Visilizumab, Vobarilizumab, Volociximab, Vorsetuzumab mafodotin, Votumumab, Xentuzumab, Zalutumumab, Zanolimumab, Zatuximab, Ziralimumab, and Zolimomab aritox and combinations thereof.

Examples of vaccines developed for viral diseases include, but are not limited to: Hepatitis A vaccine, Hepatitis B vaccine, Hepatitis E vaccine, HPV vaccine, Influenza vaccine, Japanese encephalitis vaccine, MMR vaccine, MMRV vaccine, Polio vaccine, Rabies vaccine, Rotavirus vaccine, Varicella vaccine, Shingles vaccine, Smallpox vaccine, Yellow Fever vaccine, Adenovirus vaccine, Coxsackie B virus vaccine, Cytomegalovirus vaccine, Dengue vaccine for humans, Eastern Equine encephalitis virus vaccine for humans, Ebola vaccine, Enterovirus 71 vaccine, Epstein-Barr vaccine, Hepatitis C vaccine, HIV vaccine, HTLV-1 T-lymphotropic leukemia vaccine for humans, Marburg virus disease vaccine, Norovirus vaccine, Respiratory syncytial virus vaccine for humans, Severe acute respiratory syndrome (SARS) vaccine, West Nile virus vaccine for humans; Examples of bacterial diseases include but are not limited to: Anthrax vaccines, DPT vaccine, Q fever vaccine, Hib vaccine, Tuberculosis (BCG) vaccine, Meningococcal vaccine, Typhoid vaccine, Pneumococcal conjugate vaccine, Pneumococcal polysaccharide vaccine, Cholera vaccine, Caries vaccine, Ehrlichiosis vaccine, Leprosy vaccine, Lyme disease vaccine, Staphylococcus aureus vaccine, Streptococcus pyogenes vaccine, Syphilis vaccine, Tularemia vaccine, and Yersinia pestis vaccine; Examples of parasitic diseases include, but are not limited to: Malaria vaccine, Schistosomiasis vaccine, Chagas disease vaccine, Hookworm vaccine, Onchocerciasis river blindness vaccine for humans, Trypanosomiasis vaccine, and Visceral leishmaniasis vaccine; Examples of non-infectious diseases include, but are not limited to: Alzheimer's disease amyloid protein vaccine, Breast cancer vaccine, Ovarian cancer vaccine, Prostate cancer vaccine, and Talimogene laherparepvec (T-VEC); also vaccines including, but not limited to the following trade names: ACAM2000, ActHIB, Adacel, Afluria, AFLURIA QUADRIVALENT, Agriflu, BCG Vaccine, BEXSERO, Biothrax, Boostrix, Cervarix, Comvax, DAPTACEL, DECAVAC, Engerix-B, FLUAD, Fluarix, Fluarix Quadrivalent, Flublok, Flucelvax, Flucelvax Quadrivalent, FluLaval, FluMist, FluMist Quadrivalent, Fluvirin, Fluzone Quadrivalent, Fluzone, Fluzone High-Dose and Fluzone Intradermal, Gardasil, Gardasil 9, Havrix, Hiberix, Imovax, Infanrix, IPOL, Ixiaro, JE-Vax, KINRIX, Menactra, MenHibrix, Menomune-A/C/Y/W-135, Menveo, M-M-R II, M-M-Vax, Pediarix, PedvaxHIB, Pentacel, Pneumovax 23, Poliovax, Prevnar, Prevnar 13, ProQuad, Quadracel, Quadrivalent, RabAvert, Recombivax HB, ROTARIX, RotaTeq, TENIVAC, TICE BCG, Tripedia, TRUMENBA, Twinrix, TYPHIM Vi, VAQTA, Varivax, Vaxchora, Vivotif, YF-Vax, Zostavax, and combinations thereof.

Examples of injectable drugs include, but are not limited to: Ablavar (Gadofosveset Trisodium Injection), Abarelix Depot, Abobotulinumtoxin A Injection (Dysport), ABT-263, ABT-869, ABX-EFG, Accretropin (Somatropin Injection), Acetadote (Acetylcysteine Injection), Acetazolamide Injection (Acetazolamide Injection), Acetylcysteine Injection (Acetadote), Actemra (Tocilizumab Injection), Acthrel (Corticorelin Ovine Triflutate for Injection), Actummune, Activase, Acyclovir for Injection (Zovirax Injection), Adacel, Adalimumab, Adenoscan (Adenosine Injection), Adenosine Injection (Adenoscan), Adrenaclick, AdreView (Iobenguane 1123 Injection for Intravenous Use), Afluria, Ak-Fluor (Fluorescein Injection), Aldurazyme (Laronidase), Alglucerase Injection (Ceredase), Alkeran Injection (Melphalan Hcl Injection), Allopurinol Sodium for Injection (Aloprim), Aloprim (Allopurinol Sodium for Injection), Alprostadil, Alsuma (Sumatriptan Injection), ALTU-238, Amino Acid Injections, Aminosyn, Apidra, Apremilast, Alprostadil Dual Chamber System for Injection (Caverject Impulse), AMG 009, AMG 076, AMG 102, AMG 108, AMG 114, AMG 162, AMG 220, AMG 221, AMG 222, AMG 223, AMG 317, AMG 379, AMG 386, AMG 403, AMG 477, AMG 479, AMG 517, AMG 531, AMG 557, AMG 623, AMG 655, AMG 706, AMG 714, AMG 745, AMG 785, AMG 811, AMG 827, AMG 837, AMG 853, AMG 951, Amiodarone HCl Injection (Amiodarone HCl Injection), Amobarbital Sodium Injection (Amytal Sodium), Amytal Sodium (Amobarbital Sodium Injection), Anakinra, Anti-Abeta, Anti-Beta7, Anti-Beta20, Anti-CD4, Anti-CD20, Anti-CD40, Anti-IFNalpha, Anti-IL13, Anti-OX40L, Anti-oxLDS, Anti-NGF, Anti-NRP1, Arixtra, Amphadase (Hyaluronidase Inj), Ammonul (Sodium Phenylacetate and Sodium Benzoate Injection), Anaprox, Anzemet Injection (Dolasetron Mesylate Injection), Apidra (Insulin Glulisine [rDNA origin] Inj), Apomab, Aranesp (darbepoetin alfa), Argatroban (Argatroban Injection), Arginine Hydrochloride Injection (R-Gene 10, Aristocort, Aristospan, Arsenic Trioxide Injection (Trisenox), Articane HCl and Epinephrine Injection (Septocaine), Arzerra (Ofatumumab Injection), Asclera (Polidocanol Injection), Ataluren, Ataluren-DMD, Atenolol Inj (Tenormin I.V. Injection), Atracurium Besylate Injection (Atracurium Besylate Injection), Avastin, Azactam Injection (Aztreonam Injection), Azithromycin (Zithromax Injection), Aztreonam Injection (Azactam Injection), Baclofen Injection (Lioresal Intrathecal), Bacteriostatic Water (Bacteriostatic Water for Injection), Baclofen Injection (Lioresal Intrathecal), Bal in Oil Ampules (Dimercarprol Injection), BayHepB, BayTet, Benadryl, Bendamustine Hydrochloride Injection (Treanda), Benztropine Mesylate Injection (Cogentin), Betamethasone Injectable Suspension (Celestone Soluspan), Bexxar, Bicillin C-R 900/300 (Penicillin G Benzathine and Penicillin G Procaine Injection), Blenoxane (Bleomycin Sulfate Injection), Bleomycin Sulfate Injection (Blenoxane), Boniva Injection (Ibandronate Sodium Injection), Botox Cosmetic (OnabotulinumtoxinA for Injection), BR3-FC, Bravelle (Urofollitropin Injection), Bretylium (Bretylium Tosylate Injection), Brevital Sodium (Methohexital Sodium for Injection), Brethine, Briobacept, BTT-1023, Bupivacaine HCl, Byetta, Ca-DTPA (Pentetate Calcium Trisodium Inj), Cabazitaxel Injection (Jevtana), Caffeine Alkaloid (Caffeine and Sodium Benzoate Injection), Calcijex Injection (Calcitrol), Calcitrol (Calcijex Injection), Calcium Chloride (Calcium Chloride Injection 10%), Calcium Disodium Versenate (Edetate Calcium Disodium Injection), Campath (Altemtuzumab), Camptosar Injection (Irinotecan Hydrochloride), Canakinumab Injection (Ilaris), Capastat Sulfate (Capreomycin for Injection), Capreomycin for Injection (Capastat Sulfate), Cardiolite (Prep kit for Technetium Tc99 Sestamibi for Injection), Carticel, Cathflo, Cefazolin and Dextrose for Injection (Cefazolin Injection), Cefepime Hydrochloride, Cefotaxime, Ceftriaxone, Cerezyme, Carnitor Injection, Caverject, Celestone Soluspan, Celsior, Cerebyx (Fosphenytoin Sodium Injection), Ceredase (Alglucerase Injection), Ceretec (Technetium Tc99m Exametazime Injection), Certolizumab, CF-101, Chloramphenicol Sodium Succinate (Chloramphenicol Sodium Succinate Injection), Chloramphenicol Sodium Succinate Injection (Chloramphenicol Sodium Succinate), Cholestagel (Colesevelam HCL), Choriogonadotropin Alfa Injection (Ovidrel), Cimzia, Cisplatin (Cisplatin Injection), Clolar (Clofarabine Injection), Clomiphine Citrate, Clonidine Injection (Duraclon), Cogentin (Benztropine Mesylate Injection), Colistimethate Injection (Coly-Mycin M), Coly-Mycin M (Colistimethate Injection), Compath, Conivaptan Hcl Injection (Vaprisol), Conjugated Estrogens for Injection (Premarin Injection), Copaxone, Corticorelin Ovine Triflutate for Injection (Acthrel), Corvert (Ibutilide Fumarate Injection), Cubicin (Daptomycin Injection), CF-101, Cyanokit (Hydroxocobalamin for Injection), Cytarabine Liposome Injection (DepoCyt), Cyanocobalamin, Cytovene (ganciclovir), D.H.E. 45, Dacetuzumab, Dacogen (Decitabine Injection), Dalteparin, Dantrium IV (Dantrolene Sodium for Injection), Dantrolene Sodium for Injection (Dantrium IV), Daptomycin Injection (Cubicin), Darbepoietin Alfa, DDAVP Injection (Desmopressin Acetate Injection), Decavax, Decitabine Injection (Dacogen), Dehydrated Alcohol (Dehydrated Alcohol Injection), Denosumab Injection (Prolia), Delatestryl, Delestrogen, Delteparin Sodium, Depacon (Valproate Sodium Injection), Depo Medrol (Methylprednisolone Acetate Injectable Suspension), DepoCyt (Cytarabine Liposome Injection), DepoDur (Morphine Sulfate XR Liposome Injection), Desmopressin Acetate Injection (DDAVP Injection), Depo-Estradiol, Depo-Provera 104 mg/ml, Depo-Provera 150 mg/ml, Depo-Testosterone, Dexrazoxane for Injection, Intravenous Infusion Only (Totect), Dextrose/Electrolytes, Dextrose and Sodium Chloride Inj (Dextrose 5% in 0.9% Sodium Chloride), Dextrose, Diazepam Injection (Diazepam Injection), Digoxin Injection (Lanoxin Injection), Dilaudid-HP (Hydromorphone Hydrochloride Injection), Dimercarprol Injection (Bal in Oil Ampules), Diphenhydramine Injection (Benadryl Injection), Dipyridamole Injection (Dipyridamole Injection), DMOAD, Docetaxel for Injection (Taxotere), Dolasetron Mesylate Injection (Anzemet Injection), Doribax (Doripenem for Injection), Doripenem for Injection (Doribax), Doxercalciferol Injection (Hectorol Injection), Doxil (Doxorubicin Hcl Liposome Injection), Doxorubicin Hcl Liposome Injection (Doxil), Duraclon (Clonidine Injection), Duramorph (Morphine Injection), Dysport (Abobotulinumtoxin A Injection), Ecallantide Injection (Kalbitor), EC-Naprosyn (naproxen), Edetate Calcium Disodium Injection (Calcium Disodium Versenate), Edex (Alprostadil for Injection), Engerix, Edrophonium Injection (Enlon), Eliglustat Tartate, Eloxatin (Oxaliplatin Injection), Emend Injection (Fosaprepitant Dimeglumine Injection), Enalaprilat Injection (Enalaprilat Injection), Enlon (Edrophonium Injection), Enoxaparin Sodium Injection (Lovenox), Eovist (Gadoxetate Disodium Injection), Enbrel (etanercept), Enoxaparin, Epicel, Epinepherine, Epipen, Epipen Jr., Epratuzumab, Erbitux, Ertapenem Injection (Invanz), Erythropoieten, Essential Amino Acid Injection (Nephramine), Estradiol Cypionate, Estradiol Valerate, Etanercept, Exenatide Injection (Byetta), Evlotra, Fabrazyme (Adalsidase beta), Famotidine Injection, FDG (Fludeoxyglucose F 18 Injection), Feraheme (Ferumoxytol Injection), Feridex I.V. (Ferumoxides Injectable Solution), Fertinex, Ferumoxides Injectable Solution (Feridex I.V.), Ferumoxytol Injection (Feraheme), Flagyl Injection (Metronidazole Injection), Fluarix, Fludara (Fludarabine Phosphate), Fludeoxyglucose F 18 Injection (FDG), Fluorescein Injection (Ak-Fluor), Follistim AQ Cartridge (Follitropin Beta Injection), Follitropin Alfa Injection (Gonal-f RFF), Follitropin Beta Injection (Follistim AQ Cartridge), Folotyn (Pralatrexate Solution for Intravenous Injection), Fondaparinux, Forteo (Teriparatide (rDNA origin) Injection), Fostamatinib, Fosaprepitant Dimeglumine Injection (Emend Injection), Foscarnet Sodium Injection (Foscavir), Foscavir (Foscarnet Sodium Injection), Fosphenytoin Sodium Injection (Cerebyx), Fospropofol Disodium Injection (Lusedra), Fragmin, Fuzeon (enfuvirtide), GA101, Gadobenate Dimeglumine Injection (Multihance), Gadofosveset Trisodium Injection (Ablavar), Gadoteridol Injection Solution (ProHance), Gadoversetamide Injection (OptiMARK), Gadoxetate Disodium Injection (Eovist), Ganirelix (Ganirelix Acetate Injection), Gardasil, GC1008, GDFD, Gemtuzumab Ozogamicin for Injection (Mylotarg), Genotropin, Gentamicin Injection, GENZ-112638, Golimumab Injection (Simponi Injection), Gonal-f RFF (Follitropin Alfa Injection), Granisetron Hydrochloride (Kytril Injection), Gentamicin Sulfate, Glatiramer Acetate, Glucagen, Glucagon, HAE1, Haldol (Haloperidol Injection), Havrix, Hectorol Injection (Doxercalciferol Injection), Hedgehog Pathway Inhibitor, Heparin, Herceptin, hG-CSF, Humalog, Human Growth Hormone, Humatrope, HuMax, Humegon, Humira, Humulin, Ibandronate Sodium Injection (Boniva Injection), Ibuprofen Lysine Injection (NeoProfen), Ibutilide Fumarate Injection (Corvert), Idamycin PFS (Idarubicin Hydrochloride Injection), Idarubicin Hydrochloride Injection (Idamycin PFS), Ilaris (Canakinumab Injection), Imipenem and Cilastatin for Injection (Primaxin I.V.), Imitrex, Incobotulinumtoxin A for Injection (Xeomin), Increlex (Mecasermin [rDNA origin] Injection), Indocin IV (Indomethacin Inj), Indomethacin Inj (Indocin IV), Infanrix, Innohep, Insulin, Insulin Aspart [rDNA origin] Inj (NovoLog), Insulin Glargine [rDNA origin] Injection (Lantus), Insulin Glulisine [rDNA origin] Inj (Apidra), Interferon alfa-2b, Recombinant for Injection (Intron A), Intron A (Interferon alfa-2b, Recombinant for Injection), Invanz (Ertapenem Injection), Invega Sustenna (Paliperidone Palmitate Extended-Release Injectable Suspension), Invirase (saquinavir mesylate), lobenguane 1123 Injection for Intravenous Use (AdreView), Iopromide Injection (Ultravist), loversol Injection (Optiray Injection), Iplex (Mecasermin Rinfabate [rDNA origin] Injection), Iprivask, Irinotecan Hydrochloride (Camptosar Injection), Iron Sucrose Injection (Venofer), Istodax (Romidepsin for Injection), Itraconazole Injection (Sporanox Injection), Jevtana (Cabazitaxel Injection), Jonexa, Kalbitor (Ecallantide Injection), KCL in D5NS (Potassium Chloride in 5% Dextrose and Sodium Chloride Injection), KCL in D5W, KCL in NS, Kenalog 10 Injection (Triamcinolone Acetonide Injectable Suspension), Kepivance (Palifermin), Keppra Injection (Levetiracetam), Keratinocyte, KFG, Kinase Inhibitor, Kineret (Anakinra), Kinlytic (Urokinase Injection), Kinrix, Klonopin (clonazepam), Kytril Injection (Granisetron Hydrochloride), lacosamide Tablet and Injection (Vimpat), Lactated Ringer's, Lanoxin Injection (Digoxin Injection), Lansoprazole for Injection (Prevacid I.V.), Lantus, Leucovorin Calcium (Leucovorin Calcium Injection), Lente (L), Leptin, Levemir, Leukine Sargramostim, Leuprolide Acetate, Levothyroxine, Levetiracetam (Keppra Injection), Lovenox, Levocarnitine Injection (Carnitor Injection), Lexiscan (Regadenoson Injection), Lioresal Intrathecal (Baclofen Injection), Liraglutide [rDNA] Injection (Victoza), Lovenox (Enoxaparin Sodium Injection), Lucentis (Ranibizumab Injection), Lumizyme, Lupron (Leuprolide Acetate Injection), Lusedra (Fospropofol Disodium Injection), Maci, Magnesium Sulfate (Magnesium Sulfate Injection), Mannitol Injection (Mannitol IV), Marcaine (Bupivacaine Hydrochloride and Epinephrine Injection), Maxipime (Cefepime Hydrochloride for Injection), MDP Multidose Kit of Technetium Injection (Technetium Tc99m Medronate Injection), Mecasermin [rDNA origin] Injection (Increlex), Mecasermin Rinfabate [rDNA origin] Injection (Iplex), Melphalan Hcl Injection (Alkeran Injection), Methotrexate, Menactra, Menopur (Menotropins Injection), Menotropins for Injection (Repronex), Methohexital Sodium for Injection (Brevital Sodium), Methyldopate Hydrochloride Injection, Solution (Methyldopate Hcl), Methylene Blue (Methylene Blue Injection), Methylprednisolone Acetate Injectable Suspension (Depo Medrol), MetMab, Metoclopramide Injection (Reglan Injection), Metrodin (Urofollitropin for Injection), Metronidazole Injection (Flagyl Injection), Miacalcin, Midazolam (Midazolam Injection), Mimpara (Cinacalet), Minocin Injection (Minocycline Inj), Minocycline Inj (Minocin Injection), Mipomersen, Mitoxantrone for Injection Concentrate (Novantrone), Morphine Injection (Duramorph), Morphine Sulfate XR Liposome Injection (DepoDur), Morrhuate Sodium (Morrhuate Sodium Injection), Motesanib, Mozobil (Plerixafor Injection), Multihance (Gadobenate Dimeglumine Injection), Multiple Electrolytes and Dextrose Injection, Multiple Electrolytes Injection, Mylotarg (Gemtuzumab Ozogamicin for Injection), Myozyme (Alglucosidase alfa), Nafcillin Injection (Nafcillin Sodium), Nafcillin Sodium (Nafcillin Injection), Naltrexone XR Inj (Vivitrol), Naprosyn (naproxen), NeoProfen (Ibuprofen Lysine Injection), Nandrol Decanoate, Neostigmine Methylsulfate (Neostigmine Methylsulfate Injection), NEO-GAA, NeoTect (Technetium Tc 99m Depreotide Injection), Nephramine (Essential Amino Acid Injection), Neulasta (pegfilgrastim), Neupogen (Filgrastim), Novolin, Novolog, NeoRecormon, Neutrexin (Trimetrexate Glucuronate Inj), NPH (N), Nexterone (Amiodarone HCl Injection), Norditropin (Somatropin Injection), Normal Saline (Sodium Chloride Injection), Novantrone (Mitoxantrone for Injection Concentrate), Novolin 70/30 Innolet (70% NPH, Human Insulin Isophane Suspension and 30% Regular, Human Insulin Injection), NovoLog (Insulin Aspart [rDNA origin] Inj), Nplate (romiplostim), Nutropin (Somatropin (rDNA origin) for Inj), Nutropin AQ, Nutropin Depot (Somatropin (rDNA origin) for Inj), Octreotide Acetate Injection (Sandostatin LAR), Ocrelizumab, Ofatumumab Injection (Arzerra), Olanzapine Extended Release Injectable Suspension (Zyprexa Relprevv), Omnitarg, Omnitrope (Somatropin [rDNA origin] Injection), Ondansetron Hydrochloride Injection (Zofran Injection), OptiMARK (Gadoversetamide Injection), Optiray Injection (loversol Injection), Orencia, Osmitrol Injection in Aviva (Mannitol Injection in Aviva Plastic Vessel 250), Osmitrol Injection in Viaflex (Mannitol Injection in Viaflex Plastic Vessel 250), Osteoprotegrin, Ovidrel (Choriogonadotropin Alfa Injection), Oxacillin (Oxacillin for Injection), Oxaliplatin Injection (Eloxatin), Oxytocin Injection (Pitocin), Paliperidone Palmitate Extended-Release Injectable Suspension (Invega Sustenna), Pamidronate Disodium Injection (Pam idronate Disodium Injection), Panitumumab Injection for Intravenous Use (Vectibix), Papaverine Hydrochloride Injection (Papaverine Injection), Papaverine Injection (Papaverine Hydrochloride Injection), Parathyroid Hormone, Paricalcitol Injection Fliptop Vial (Zemplar Injection), PARP Inhibitor, Pediarix, PEGlntron, Peginterferon, Pegfilgrastim, Penicillin G Benzathine and Penicillin G Procaine, Pentetate Calcium Trisodium Inj (Ca-DTPA), Pentetate Zinc Trisodium Injection (Zn-DTPA), Pepcid Injection (Famotidine Injection), Pergonal, Pertuzumab, Phentolamine Mesylate (Phentolamine Mesylate for Injection), Physostigmine Salicylate (Physostigmine Salicylate (injection)), Physostigmine Salicylate (injection) (Physostigmine Salicylate), Piperacillin and Tazobactam Injection (Zosyn), Pitocin (Oxytocin Injection), Plasma-Lyte 148 (Multiple Electrolytes Inj), Plasma-Lyte 56 and Dextrose (Multiple Electrolytes and Dextrose Injection in Viaflex, Plastic Vessel 250), PlasmaLyte, Plerixafor Injection (Mozobil), Polidocanol Injection (Asclera), Potassium Chloride, Pralatrexate Solution for Intravenous Injection (Folotyn), Pramlintide Acetate Injection (Symlin), Premarin Injection (Conjugated Estrogens for Injection), Prep kit for Technetium Tc99 Sestamibi for Injection (Cardiolite), Prevacid I.V. (Lansoprazole for Injection), Primaxin I.V. (Imipenem and Cilastatin for Injection), Prochymal, Procrit, Progesterone, ProHance (Gadoteridol Injection Solution), Prolia (Denosumab Injection), Promethazine HCl Injection (Promethazine Hydrochloride Injection), Propranolol Hydrochloride Injection (Propranolol Hydrochloride Injection), Quinidine Gluconate Injection (Quinidine Injection), Quinidine Injection (Quinidine Gluconate Injection), R-Gene 10 (Arginine Hydrochloride Injection), Ranibizumab Injection (Lucentis), Ranitidine Hydrochloride Injection (Zantac Injection), Raptiva, Reclast (Zoledronic Acid Injection), Recombivarix HB, Regadenoson Injection (Lexiscan), Reglan Injection (Metoclopramide Injection), Remicade, Renagel, Renvela (Sevelamer Carbonate), Repronex (Menotropins for Injection), Retrovir IV (Zidovudine Injection), rhApo2L/TRAIL, Ringer's and 5% Dextrose Injection (Ringers in Dextrose), Ringer's Injection (Ringers Injection), Rituxan, Rituximab, Rocephin (ceftriaxone), Rocuronium Bromide Injection (Zemuron), Roferon-A (interferon alfa-2a), Romazicon (flumazenil), Romidepsin for Injection (Istodax), Saizen (Somatropin Injection), Sandostatin LAR (Octreotide Acetate Injection), Sclerostin Ab, Sensipar (cinacalcet), Sensorcaine (Bupivacaine HCl Injections), Septocaine (Articane HCl and Epinephrine Injection), Serostim LQ (Somatropin (rDNA origin) Injection), Simponi Injection (Golimumab Injection), Sodium Acetate (Sodium Acetate Injection), Sodium Bicarbonate (Sodium Bicarbonate 5% Injection), Sodium Lactate (Sodium Lactate Injection in AVIVA), Sodium Phenylacetate and Sodium Benzoate Injection (Ammonul), Somatropin (rDNA origin) for Inj (Nutropin), Sporanox Injection (Itraconazole Injection), Stelara Injection (Ustekinumab), Stemgen, Sufenta (Sufentanil Citrate Injection), Sufentanil Citrate Injection (Sufenta), Sumavel, Sumatriptan Injection (Alsuma), Symlin, Symlin Pen, Systemic Hedgehog Antagonist, Synvisc-One (Hylan G-F 20 Single Intra-articular Injection), Tarceva, Taxotere (Docetaxel for Injection), Technetium Tc 99m, Telavancin for Injection (Vibativ), Temsirolimus Injection (Torisel), Tenormin I.V. Injection (Atenolol Inj), Teriparatide (rDNA origin) Injection (Forteo), Testosterone Cypionate, Testosterone Enanthate, Testosterone Propionate, Tev-Tropin (Somatropin, rDNA Origin, for Injection), tgAAC94, Thallous Chloride, Theophylline, Thiotepa (Thiotepa Injection), Thymoglobulin (Anti-Thymocyte Globulin (Rabbit), Thyrogen (Thyrotropin Alfa for Injection), Ticarcillin Disodium and Clavulanate Potassium Galaxy (Timentin Injection), Tigan Injection (Trimethobenzamide Hydrochloride Injectable), Timentin Injection (Ticarcillin Disodium and Clavulanate Potassium Galaxy), TNKase, Tobramycin Injection (Tobramycin Injection), Tocilizumab Injection (Actemra), Torisel (Temsirolimus Injection), Totect (Dexrazoxane for Injection, Intravenous Infusion Only), Trastuzumab-DM1, Travasol (Amino Acids (Injection)), Treanda (Bendamustine Hydrochloride Injection), Trelstar (Triptorelin Pamoate for Injectable Suspension), Triamcinolone Acetonide, Triamcinolone Diacetate, Triamcinolone Hexacetonide Injectable Suspension (Aristospan Injection 20 mg), Triesence (Triamcinolone Acetonide Injectable Suspension), Trimethobenzamide Hydrochloride Injectable (Tigan Injection), Trimetrexate Glucuronate Inj (Neutrexin), Triptorelin Pamoate for Injectable Suspension (Trelstar), Twinject, Trivaris (Triamcinolone Acetonide Injectable Suspension), Trisenox (Arsenic Trioxide Injection), Twinrix, Typhoid Vi, Ultravist (Iopromide Injection), Urofollitropin for Injection (Metrodin), Urokinase Injection (Kinlytic), Ustekinumab (Stelara Injection), Ultralente (U), Valium (diazepam), Valproate Sodium Injection (Depacon), Valtropin (Somatropin Injection), Vancomycin Hydrochloride (Vancomycin Hydrochloride Injection), Vancomycin Hydrochloride Injection (Vancomycin Hydrochloride), Vaprisol (Conivaptan Hcl Injection), VAQTA, Vasovist (Gadofosveset Trisodium Injection for Intravenous Use), Vectibix (Panitumumab Injection for Intravenous Use), Venofer (Iron Sucrose Injection), Verteporfin Inj (Visudyne), Vibativ (Telavancin for Injection), Victoza (Liraglutide [rDNA] Injection), Vimpat (lacosamide Tablet and Injection), Vinblastine Sulfate (Vinblastine Sulfate Injection), Vincasar PFS (Vincristine Sulfate Injection), Victoza, Vincristine Sulfate (Vincristine Sulfate Injection), Visudyne (Verteporfin Inj), Vitamin B-12, Vivitrol (Naltrexone XR Inj), Voluven (Hydroxyethyl Starch in Sodium Chloride Injection), Xeloda, Xenical (orlistat), Xeomin (Incobotulinumtoxin A for Injection), Xolair, Zantac Injection (Ranitidine Hydrochloride Injection), Zemplar Injection (Paricalcitol Injection Fliptop Vial), Zemuron (Rocuronium Bromide Injection), Zenapax (daclizumab), Zevalin, Zidovudine Injection (Retrovir IV), Zithromax Injection (Azithromycin), Zn-DTPA (Pentetate Zinc Trisodium Injection), Zofran Injection (Ondansetron Hydrochloride Injection), Zingo, Zoledronic Acid for Inj (Zometa), Zoledronic Acid Injection (Reclast), Zometa (Zoledronic Acid for Inj), Zosyn (Piperacillin and Tazobactam Injection), Zyprexa Relprevv (Olanzapine Extended Release Injectable Suspension) and combinations thereof.

### Notice

The invention of this application has been described above both generically and with regard to specific embodiments. It will be apparent to those skilled in the art that various modifications and variations can be made in the embodiments without departing from the scope of the disclosure. Thus, it is intended that the embodiments cover the modifications and variations of this invention provided they come within the scope of the appended claims and their equivalents.

## Claims

1. A stopper for a syringe, the stopper comprising:
a body having a leading end, a trailing end, and an outer surface extending between the leading end and the trailing end, the outer surface operable to sealingly and slidably engage a barrel of a syringe;
wherein the body of the stopper has an inner surface and a pocket having a first end and a second end, the pocket being defined by the inner surface and the body further having an opening into the second end of the pocket that is formed in the trailing end of the body;
wherein the pocket includes a capture portion having a first diameter, a relief portion having a second diameter, and a coupling portion between the capture portion and the relief portion that has a third diameter that is less than the first and second diameters;
wherein the inner surface of the body includes one or more coupling projections corresponding to the coupling portion of the pocket.

2. The stopper of claim 1, wherein the one or more coupling projections includes a circumferential rib, and optionally the circumferential rib extends continuously about a circumference of the pocket.

3. The stopper of claim 1 or claim 2, wherein the stopper has one or more of the following features:
- the one or more coupling projections includes a longitudinal rib;
- the one or more coupling projections has a leading edge and a trailing edge, and further wherein at least the leading edge is at least one of angled and rounded;
- the first diameter approximately the same as the second diameter;
- the third diameter is at least 10% smaller than the first diameter and/or the second diameter;
- the first diameter differs from the third diameter by approximately 0.3 mm; and
- the second diameter differs from the third diameter by approximately 0.3 mm.

4. A syringe comprising:
a barrel having an outer surface, an inner surface, and a receiving chamber, the inner surface bounding the receiving chamber;
a stopper positioned in the receiving chamber such that the stopper is slidably and sealingly engaged with the inner surface of the barrel, the stopper having a pocket including a capture portion having a first diameter, a relief portion having a second diameter, and a coupling portion between the capture portion and the relief portion that has a third diameter that is less than the first and second diameters, the stopper including one or more coupling projections corresponding to the coupling portion of the pocket; and
a plunger rod having a head portion, a rear portion, and a rod portion extending between the head portion and the rear portion, the head portion having a tapered crown and defining retaining feature engaged with the coupling portion of the pocket to couple the plunger rod to the stopper with the head portion received in the capture portion of the stopper.

5. The syringe of claim 4, wherein the tapered crown has a smooth surface for slidably engaging with the one or more coupling projections of the coupling portion during insertion of the plunger rod head portion into the pocket of the stopper.

6. The syringe of claims 4 or 5, wherein the stopper and the barrel define a break loose force, and the stopper and the plunger rod are configured such that the plunger rod head portion is insertable axially into the stopper with the stopper received in the barrel at an insertion force that is less than the break loose force, and optionally wherein the break loose force is from 2N to 20N.

7. The syringe of any of claims 4 to 6, wherein the stopper and the plunger rod require application of a separation force in a longitudinal direction to decouple the plunger rod from the stopper.

8. The syringe of claim 6, wherein the stopper and the plunger rod require application of a separation force in a longitudinal direction to decouple the plunger rod from the stopper and the separation force is greater than the break loose force.

9. A method of coupling a plunger rod to a stopper that has been positioned in a barrel of a syringe, the method comprising axially inserting a head portion of the plunger rod into a pocket of the stopper with an insertion force that is less than a break loose force defined between the stopper and the barrel of the syringe, wherein upon axially inserting the head portion of the plunger rod into a capture portion of the stopper the plunger rod is coupled to the stopper.

10. The method of claim 9, wherein inserting the head portion into the pocket includes sliding a tapered crown of the head portion of the plunger rod past one or more coupling projections corresponding to the coupling portion of the pocket in order to couple the plunger rod to the stopper, and optionally wherein the one or more coupling projections include one or more longitudinally extending ribs and/or one or more circumferentially extending ribs.

11. The method of claim 9 or claim 10, wherein each of the one or more coupling projections has a leading edge and a trailing edge, and further wherein at least the leading edge is at least one of angled and rounded, and further wherein inserting the head portion into the pocket includes sliding the head portion longitudinally past the leading edge of the one or more coupling projections, and optionally wherein the break loose force is from 2N to 20N.

12. The method of any of claims 9 to 11, wherein the stopper and the plunger rod require application of a separation force in a longitudinal direction to decouple the plunger rod from the stopper, and optionally wherein the separation force is greater than the break loose force.

13. A stopper for a syringe, the stopper comprising:
a body having a leading end, a trailing end, and an outer surface extending between the leading end and the trailing end, the outer surface operable to sealingly and slidably engage a barrel of a syringe;
wherein the body of the stopper has an inner surface and a pocket having a first end and a second end, the pocket being defined by the inner surface and the body further having an opening into the second end of the pocket that is formed in the trailing end of the body;
wherein the pocket includes a capture portion having a first diameter, a relief portion having a second diameter, and a coupling portion between the capture portion and the relief portion that has a third diameter that is less than the first and second diameters;
wherein the inner surface of the body includes one or more coupling recesses corresponding to the coupling portion of the pocket.

14. The stopper of claim 13, wherein the one or more coupling recesses includes a circumferential recess, and optionally wherein the circumferential recess extends continuously about a circumference of the pocket.

15. The stopper of claim 13 or claim 14, wherein the stopper has one or more of the following features:
- the one or more coupling recesses includes a longitudinal recess;
- the one or more coupling recesses has a leading edge and a trailing edge, and further wherein at least the leading edge is at least one of angled and rounded;
- the first diameter approximately the same as the second diameter;
- the third diameter is at least 10% greater than the first diameter and/or the second diameter;
- the first diameter differs from the third diameter by approximately 0.3 mm; and
- the second diameter differs from the third diameter by approximately 0.3 mm.

16. A method of coupling a plunger rod to a stopper that has been positioned in a barrel of a syringe, the method comprising axially inserting a head portion of the plunger rod into a pocket of the stopper with an insertion force that is less than a break loose force defined between the stopper and the barrel of the syringe, wherein upon axially inserting the head portion of the plunger rod into a capture portion of the stopper the plunger rod is coupled to the stopper.

17. The method of claim 16, wherein inserting the head portion into the pocket also includes sliding an enlarged segment of the head portion of the plunger rod into one or more coupling recesses corresponding to a coupling portion of the pocket in order to couple the plunger rod to the stopper,
and optionally the one or more coupling recesses includes one or more longitudinally-extending recesses and/or one or more circumferentially-extending recesses,
and further optionally each of the one or more coupling recesses has a leading edge and a trailing edge, where at least the leading edge is at least one of angled and rounded, and, inserting the head portion into the pocket includes sliding the head portion longitudinally past the leading edge of the one or more coupling recesses to seat an enlarged segment of the head portion into the one or more coupling recesses.

18. The method of claim 16 or claim 17, wherein the break loose force is from 2N to 20N and the stopper and the plunger rod require application of a separation force in a longitudinal direction to decouple the plunger rod from the stopper once the plunger rod is inserted into the stopper, and optionally the separation force is greater than the break loose force.

19. A syringe comprising:
a barrel having an outer surface, an inner surface, and a receiving chamber, the inner surface bounding the receiving chamber;
a stopper positioned in the receiving chamber such that the stopper is slidably and sealingly engaged with the inner surface of the barrel, the stopper having a pocket including a capture portion having a first diameter, a relief portion having a second diameter, and a coupling portion between the capture portion and the relief portion that has a third diameter that is greater than the first and second diameters, the stopper including one or more coupling recesses corresponding to the coupling portion of the pocket; and
a plunger rod having a head portion, a rear portion, and a rod portion extending between the head portion and the rear portion, the head portion defining retaining feature engaged with the coupling recess of the pocket to couple the plunger rod to the stopper with the retaining feature received in the one or more coupling recesses of the stopper.

20. The syringe of claim 19, wherein the stopper and the barrel define a break loose force, and the stopper and the plunger rod are configured such that the plunger rod head portion is insertable axially into the stopper with the stopper received in the barrel at an insertion force that is less than the break loose force, and optionally the break loose force is from 2N to 20N.

21. The syringe of claim 19 or claim 20, wherein the stopper and the plunger rod require application of a separation force in a longitudinal direction to decouple the plunger rod from the stopper.

22. The syringe of claim 20, wherein the stopper and the plunger rod require application of a separation force in a longitudinal direction to decouple the plunger rod from the stopper and the separation force is greater than the break loose force.
